(19) Europäisches Patentamt
European Patent Office
Office européen des brevets

(11) EP 0 904 268 B1

(12) **EUROPÄISCHE PATENTSCHRIFT**

(45) Veröffentlichungstag und Bekanntmachung des Hinweises auf die Patenterteilung:
**04.12.2002 Patentblatt 2002/49**

(21) Anmeldenummer: **97923924.1**

(22) Anmeldetag: **16.05.1997**

(51) Int Cl.[7]: **C07D 273/00**, C07D 307/82, C07D 307/83

(86) Internationale Anmeldenummer:
**PCT/EP97/02526**

(87) Internationale Veröffentlichungsnummer:
**WO 97/046542 (11.12.1997 Gazette 1997/53)**

(54) **VERFAHREN ZUR HERSTELLUNG VON 3-(1-HYDROXYPHENYL-1-ALKOXIMINOMETHYL)DIOXAZINEN**

PROCESSES FOR PREPARING 3-(1-HYDROXYPHENYL-1-ALKOXIMINOMETHYL)DIOXAZINES

PROCEDES DE PREPARATION DE 3-(1-HYDROXYPHENYL-1-ALCOXIMINOMETHYLE)DIOXAZINES

(84) Benannte Vertragsstaaten:
**AT BE CH DE DK ES FR GB IT LI NL**

(30) Priorität: **30.05.1996 DE 19621696**
**09.12.1996 DE 19651034**
**19.02.1997 DE 19706399**

(43) Veröffentlichungstag der Anmeldung:
**31.03.1999 Patentblatt 1999/13**

(60) Teilanmeldung:
**01122525.7 / 1 188 753**

(73) Patentinhaber: **Bayer CropScience AG**
**40789 Monheim (DE)**

(72) Erfinder:
- **GAYER, Herbert**
  **D-40789 Monheim (DE)**
- **GALLENKAMP, Bernd**
  **D-42113 Wuppertal (DE)**
- **GERDES, Peter**
  **D-52080 Aachen (DE)**
- **HEINEMANN, Ulrich**
  **D-42799 Leichlingen (DE)**
- **KRÜGER, Bernd-Wieland**
  **D-51467 Bergisch Gladbach (DE)**
- **LANTZSCH, Reinhard**
  **D-42115 Wuppertal (DE)**
- **SEITZ, Thomas**
  **D-40764 Langenfeld (DE)**
- **STELZER, Uwe**
  **D-51399 Burscheid (DE)**

(56) Entgegenhaltungen:
**DE-A- 4 408 005**

- **PROC. INDIAN ACAD. SCI., SECT. A, Bd. 83A, Nr. 6, 1976, Seiten 238-42, XP002039743 K. V. RAO ET AL.: "Reactivity of 2-Hydroxy-omega-nitroacetophenones: synthesis of 2-oximinocoumaranones" in der Anmeldung erwähnt**
- **INDIAN JOURNAL OF CHEMISTRY, Bd. 15B, Nr. 3, 1977, Seiten 236-7, XP002039744 K. V. RAO ET AL.: "Synthesis of 2-Methyl-3-nitrochromones"**
- **INDIAN JOURNAL OF CHEMISTRY, Bd. 11, Nr. 10, 1973, Seiten 989-90, XP002039745 O. P. JHA: "Synthesis of Abutic Acid (5,6-Dimethoxycoumarone-2,3-dicarboxylic Acid)"**
- **TETRAHEDRON LETTERS, Nr. 24, 1979, Seiten 2221-4, XP002039746 W. V. CURRAN ET AL.: "A novel conversion of chromone-3-carboxaldehyde to 5-nitro-2,3-benzofurandione-(Z)-2-oxime"**
- **EUR. J. MED. CHEM. - CHIM. THER., Bd. 19, Nr. 6, 1984, Seiten 551-4, XP002039747 S. SMATI ET AL.: "Synthèse et activités anti-inflammatoire et analgésique d'acides (2H-benzofurannone-3)-2 iminoxy alcanoiques"**
- **ARCHIV DER PHARMAZIE, Bd. 306, Nr. 2, 1973, Seiten 122-6, XP002039748 H. LOTH ET AL.: "Eine neue Synthese von 2-Acyloxyimino-cumaranonen"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 904 268 B1

Printed by Jouve, 75001 PARIS (FR)

## Beschreibung

Die vorliegende Erfindung betrifft mehrere neue Verfahren und neue Zwischenprodukte zur Herstellung von 3-(1-Hydroxyphenyl-1-alkoximinomethyl)dioxazinen, die als Vorprodukte zur Herstellung von Verbindungen mit fungiziden Eigenschaften bekannt sind (WO 95-04728).

Es ist bereits bekannt geworden, daß sich bestimmte 3-(1-Hydroxyphenyl-1-alkoximinomethyl)dioxazine ausgehend von den entsprechenden Hydroxyphenylessigsäureestern synthetisieren lassen (vgl. WO 95-04728). So erhält man z. B. (5,6-Dihydro-[1,4,2]dioxazin-3-yl)-(2-hydroxy-phenyl)-methanon-O-methyl-oxim (1), indem man Hydroxyphenylessigsäuremethylester (a) mit Dihydropyran umsetzt, den dabei entstehenden Dihydropyranylether (b) mit t-Butylnitrit in den 2-[2-(Tetrahydropyran-2-yloxy)-phenyl]-2-hydroximino-essigsäuremethylester (c) überführt, diese Verbindung mit Iodmethan zu 2-[2-(Tetrahydropyran-2-yloxy)-phenyl]-2-methoximino-essigsäuremethylester (d) alkyliert, diesen mit Hydroxylamin zu 2-[2-(Tetrahydropyran-2-yloxy)-phenyl]-2-methoximino-N-hydroxyacetamid (e) umsetzt, letzteres mit Dibromethan zu 3-{1-[2-(Tetrahydropyran-2-yloxy)-phenyl]-1-methoximino-methyl}-5,6-dihydro-1,4,2-dioxazin (f) cyclisiert, und zum Schluß die Tetrahydropyranylgruppe säurekatalysiert abspaltet. Diese Synthese kann durch das folgende Formelschema veranschaulicht werden:

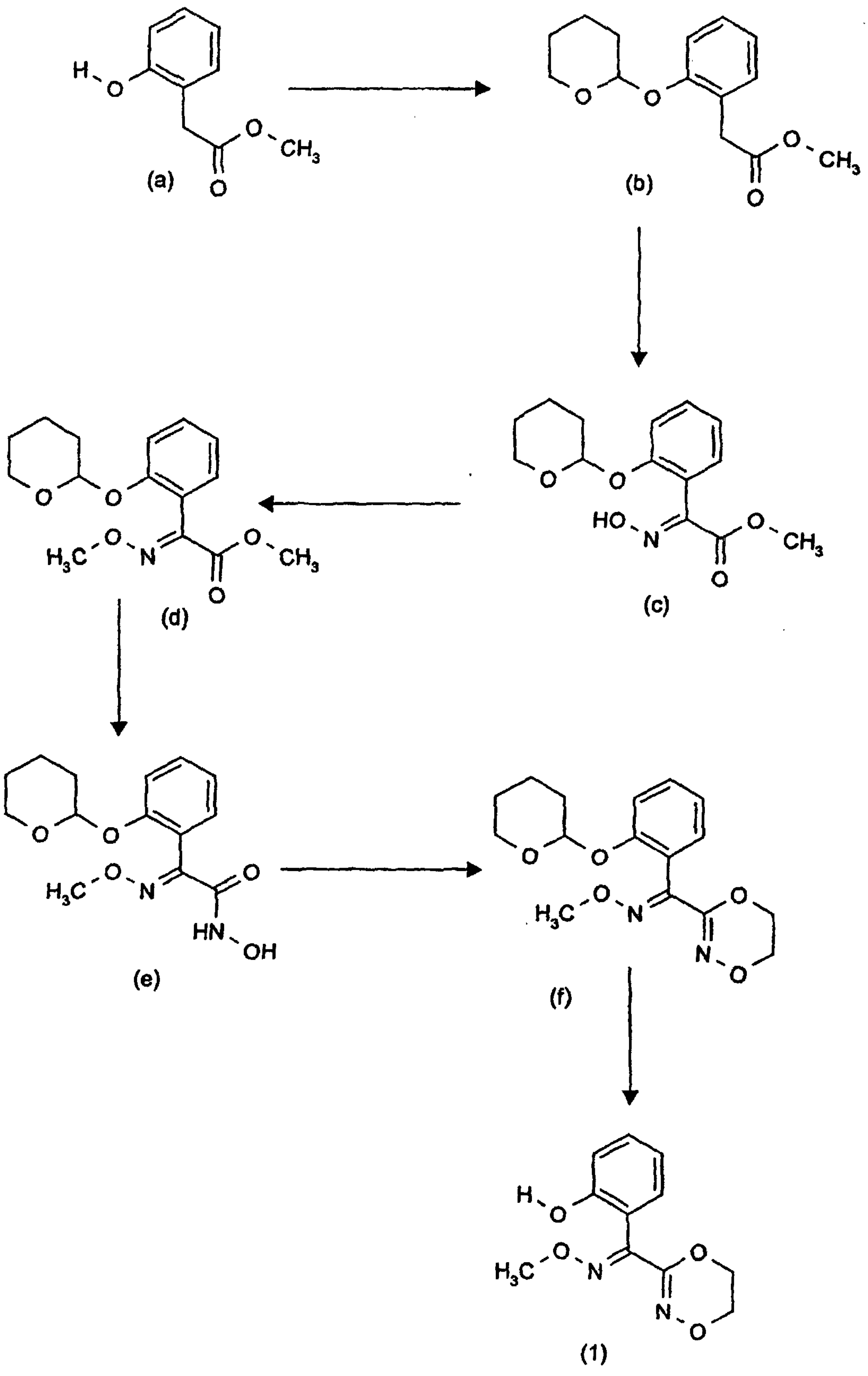

(a) (b) (c) (d) (e) (f) (1)

Ein entscheidender Nachteil dieses Verfahrens besteht darin, daß viele Reaktionsschritte mit teilweise geringen Ausbeuten erforderlich sind, was die Wirtschaftlichkeit dieses Verfahrens entscheidend beeinträchtigt.

Es wurde nun gefunden, daß man 3-(1-Hydroxyphenyl-1-alkoximinomethyl)dioxazine der allgemeinen Formel

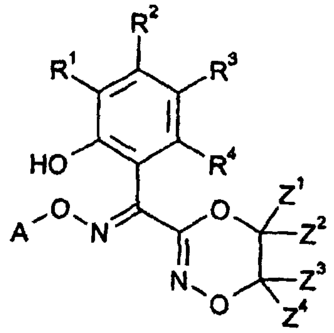

(I),

in welcher

| | |
|---|---|
| A | für Alkyl steht, |
| $R^1$, $R^2$, $R^3$ und $R^4$ | gleich oder verschieden sind und unabhängig voneinander jeweils für Wasserstoff, Halogen, Cyano, Nitro, jeweils gegebenenfalls durch Halogen substituiertes Alkyl, Alkoxy, Alkylthio, Alkylsulfinyl oder Alkylsulfonyl stehen und |
| $Z^1$, $Z^2$, $Z^3$ und $Z^4$ | gleich oder verschieden sind und unabhängig voneinander für Wasserstoff, Alkyl, Halogenalkyl oder Hydroxyalkyl stehen, oder |
| $Z^1$ und $Z^2$ oder $Z^1$ und $Z^3$ oder $Z^3$ und $Z^4$ | gemeinsam mit den jeweiligen Kohlenstoffatomen, an die sie gebunden sind, einen cycloaliphatischen Ring bilden, |

erhält, wenn man

a) O-Hydroxyethyl-O'-methyl-benzofurandiondioxime der Formel

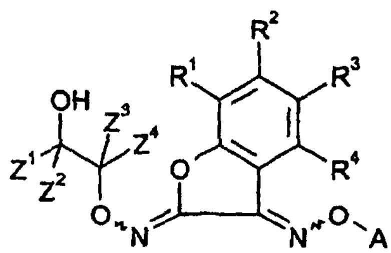

(II),

in welcher

| | |
|---|---|
| A, $R^1$, $R^2$, $R^3$, $R^4$, $Z^1$, $Z^2$, $Z^3$ und $Z^4$ | die oben angegebenen Bedeutungen haben, |

gegebenenfalls in Gegenwart eines Verdünnungsmittels und gegebenenfalls in Gegenwart einer Säure oder einer Base, umlagert.

In den Definitionen sind die gesättigten oder ungesättigten Kohlenwasserstoffketten, wie Alkyl, auch in Verknüpfung mit Heteroatomen, wie in Alkoxy oder Alkylthio, jeweils geradkettig oder verzweigt.

Nach den erfindungsgemäßen Verfahren a-c) werden bevorzugt Verbindungen der Formel (I) hergestellt, in welcher

A — für Methyl, Ethyl, n-, oder i-Propyl steht,

$R^1$, $R^2$, $R^3$ und $R^4$, — gleich oder verschieden sind und unabhängig voneinander jeweils fiir Wasserstoff, Halogen, Cyano, Nitro, jeweils gegebenenfalls durch 1 bis 5 Halogenatome substituiertes Alkyl, Alkoxy, Alkylthio, Alkylsulfinyl oder Alkylsulfonyl mit jeweils 1 bis 6 Kohlenstoffatomen, steht,

$Z^1$, $Z^2$, $Z^3$ und $Z^4$ — gleich oder verschieden sind und unabhängig voneinander für Wasserstoff, Alkyl oder Hydroxyalkyl mit 1 bis 4 Kohlenstoffatomen oder Halogenalkyl mit 1 bis 4 Kohlenstoffatomen und 1 bis 5 gleichen oder verschiedenen Halogenatomen stehen, oder

$Z^1$ und $Z^2$ oder $Z^1$ und $Z^3$ oder $Z^3$ und $Z^4$ — gemeinsam mit den jeweiligen Kohlenstoffatomen, an die sie gebunden sind, einen cycloaliphatischen Ring mit fünf, sechs oder sieben Kohlenstoffatomen bilden.

Besonders bevorzugt ist die Herstellung von Verbindungen der Formel (I), in welcher

A — für Methyl oder Ethyl steht,

$R^1$, $R^2$, $R^3$ und $R^4$ — gleich oder verschieden sind und unabhängig voneinander jeweils für Wasserstoff, Fluor, Chlor, Brom, Cyano, Nitro, Methyl, Ethyl, n-oder i-Propyl, n-, i-, s- oder t-Butyl, Methoxy, Ethoxy, n- oder i-Propoxy, Methylthio, Ethylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl stehen, und

$Z^1$, $Z^2$, $Z^3$ und $Z^4$ — gleich oder verschieden sind und unabhängig voneinander fiir Wasserstoff, Methyl, Ethyl, n- oder i-Propyl, n-, i-, s- oder t-Butyl, Hydroxymethyl, Trifluormethyl oder Trifluorethyl stehen, oder

$Z^1$ und $Z^2$ oder $Z^1$ und $Z^3$ oder $Z^3$ und $Z^4$ — gemeinsam mit den jeweiligen Kohlenstoffatomen, an die sie gebunden sind, einen cycloaliphatischen Ring mit fünf, sechs oder sieben Kohlenstoffatomen bilden.

Die zur Durchführung des erfindungsgemäßen Verfahrens a) als Ausgangsstoffe benötigten O-Hydroxyethyl-O'-methyl-benzofurandiondioxime sind durch die Formel (II) allgemein definiert. In dieser Formel (II) haben A, $R^1$, $R^2$, $R^3$, $R^4$, $Z^1$, $Z^2$, $Z^3$ und $Z^4$ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für A, $R^1$, $R^2$, $R^3$, $R^4$, $Z^1$, $Z^2$, $Z^3$ und $Z^4$ angegeben wurden.

Die Ausgangsstoffe der Formel (II) sind noch nicht bekannt und als neue Stoffe ebenfalls Gegenstand der vorliegenden Anmeldung.

Die O-Hydroxyethyl-O'-methyl-benzofurandiondioxime der Formel (II) werden erhalten, wenn man Verfahren d) O-Hydroxyethyl-benzofurandionmonooxime der Formel

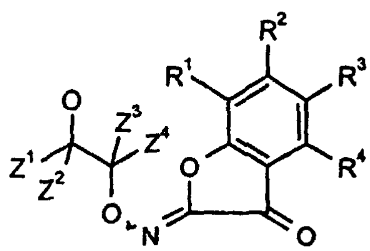

(VII)

in welcher
$R^1$, $R^2$, $R^3$, $R^4$, $Z^1$, $Z^2$, $Z^3$ und $Z^4$ die oben angegebenen Bedeutungen haben,
mit einem Alkoxyamin der Formel (IV)

$$A\text{-}O\text{-}NH_2 \qquad (IV)$$

in welcher

A die oben angegebene Bedeutung hat,

- oder einem Säureadditionskomplex davon -
gegebenenfalls in Gegenwart eines Verdünnungsmittel und gegebenenfalls in Gegenwart eines Säureakzeptors, umsetzt, oder wenn man
Verfahren e) O-Alkyl-benzofurandiondioxime der Formel

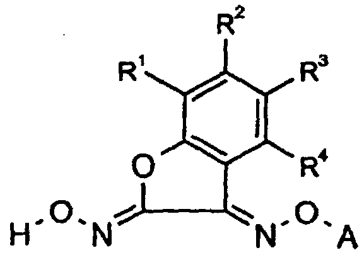
(VIII)

in welcher

A, $R^1$, $R^2$, $R^3$ und $R^4$ die oben angegebenen Bedeutungen haben,

mit einem Ethanderivat der Formel

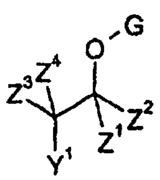
(IX)

in welcher

$Y^1$ für Halogen, Alkylsulfonyloxy, Arylsulfonyloxy oder Alkanoyloxy steht und

G für Wasserstoff steht, oder

$Y^1$ und G durch eine Einfachbindung miteinander verbunden sind, wobei

$Y^1$ für Sauerstoff steht und

G für

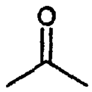

steht, oder

$Y^1$ und G gemeinsam für eine Einfachbindung stehen und

$Z^1$, $Z^2$, $Z^3$ und $Z^4$ die oben angegebenen Bedeutungen haben,

gegebenenfalls in Gegenwart eines Verdünnungsmittel, und gegebenenfalls in Gegenwart einer Base, umsetzt, oder wenn man
Verfahren f) O-Hydroxyethyl-benzofurandiondioxime der Formel

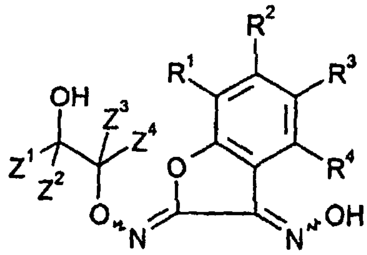

(X)

in welcher

$R^1$, $R^2$, $R^3$, $R^4$, $Z^1$, $Z^2$, $Z^3$ und $Z^4$ die oben angegebenen Bedeutungen haben,

mit einem Alkylierungsmittel der Formel (VI),
gegebenenfalls in Gegenwart eines Verdünnungsmittels, und gegebenenfalls in Gegenwart einer Base, umsetzt oder wenn man
Verfahren m) O-Oxyethyl-O'-methyl-benzofurandiondioxime der Formel

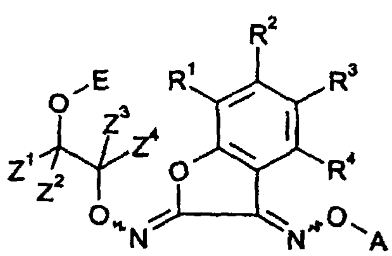

(XIII),

in welcher

A, $R^1$, $R^2$, $R^3$, $R^4$, $Z^1$, $Z^2$, $Z^3$ und $Z^4$ die oben angegebenen Bedeutungen haben und
E für eine Acylgruppe oder eine ketalische Schutzgruppe steht,

mit Wasser oder einem Alkohol, gegebenenfalls in Gegenwart eines Verdünnungsmittels und gegebenenfalls in Gegenwart einer Säure oder einer Base, umsetzt.

Die zur Durchführung des erfindungsgemäßen Verfahrens d) als Ausgangsstoffe benötigten O-Hydroxyethyl-benzofurandionmonooxime sind durch die Formel (VII) allgemein definiert. In dieser Formel (VII) haben $R^1$, $R^2$, $R^3$,

$R^4$, $Z^1$, $Z^2$, $Z^3$ und $Z^4$ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für $R^1$, $R^2$, $R^3$, $R^4$, $Z^1$, $Z^2$, $Z^3$ und $Z^4$ angegeben wurden.

Die Ausgangsstoffe der Formel (VII) sind noch nicht bekannt und als neue Stoffe ebenfalls Gegenstand der vorliegenden Anmeldung.

Die O-Hydroxyethyl-benzofurandionmonooxime der Formel (VII) werden erhalten, wenn man
Verfahren g) Benzofurandionmonooxime der Formel

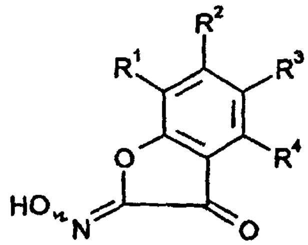

(XI)

in welcher

$R^1$, $R^2$, $R^3$ und $R^4$ die oben angegebenen Bedeutungen haben,

mit einem Ethanderivat der Formel (IX),
gegebenenfalls in Gegenwart eines Verdünnungsmittels, und gegebenenfalls in Gegenwart einer Base, umsetzt, oder wenn man
Verfahren n) O-Oxyethyl-benzofurandionmonooxime der Formel

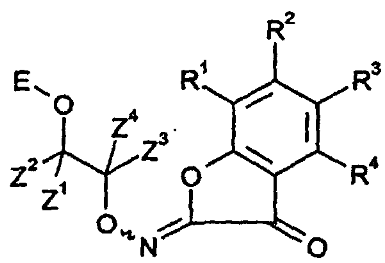

(XIV)

in welcher

E, $R^1$, $R^2$, $R^3$, $R^4$, $Z^1$, $Z^2$, $Z^3$ und $Z^4$ die oben angegebenen Bedeutungen haben,

mit Wasser oder einem Alkohol, gegebenenfalls in Gegenwart eines Verdünnungsmittels und gegebenenfalls in Gegenwart einer Säure oder einer Base, umsetzt.

Die zur Durchführung des erfindungsgemäßen Verfahrens g) als Ausgangsstoffe benötigten Benzofurandionmonooxime sind durch die Formel (XI) allgemein definiert. In dieser Formel (XI) haben $R^1$, $R^2$, $R^3$ und $R^4$ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für $R^1$, $R^2$, $R^3$ und $R^4$ angegeben wurden.

Die Benzofurandionmonooxime der Formel (XI) sind bekannt und können nach bekannten Verfahren hergestellt werden (vergleiche Beilstein, E (II) 17, 462; Mameli, G. 56, 768; Chem. Ber. 35 (1902), 1640-1646; Proc. Indian Acad. Sci. Sect. A (1976) 83A(6), 238-242)).

Die zur Durchführung des erfindungsgemäßen Verfahrens n) als Ausgangsstoffe benötigten O-Oxyethyl-benzofurandionmonooxime sind durch die Formel (XIV) allgemein definiert. In dieser Formel (XIV) haben $R^1$, $R^2$, $R^3$, $R^4$, $Z^1$, $Z^2$, $Z^3$ und $Z^4$ vorzugsweise bzw. insbesondere diejenigen Bedeutungen, die bereits im Zusammenhang mit der

Beschreibung der erfindungsgemäß herstellbaren Verbindungen der Formel (I) als bevorzugt bzw. als insbesondere bevorzugt für $R^1$, $R^2$, $R^3$, $R^4$, $Z^1$, $Z^2$, $Z^3$ und $Z^4$ angegeben wurden. E hat vorzugsweise bzw. insbesondere diejenige Bedeutung, die weiter unten im Zusammenhang mit der Beschreibung der erfindungsgemäßen Verbindungen der Formel (XIII) als bevorzugt bzw. als insbesondere bevorzugt für E angegeben wird.

Die Ausgangsstoffe der Formel (XIV) sind noch nicht bekannt und als neue Stoffe ebenfalls Gegenstand der vorliegenden Anmeldung.

Die O-Oxyethyl-benzofurandionmonooxime der Formel (XIV) werden erhalten, wenn man
Verfahren o) Benzofurandionmonooxime der Formel (XI) mit einem Ethanolderivat der Formel

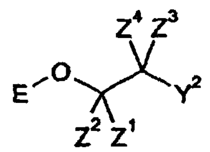

(XV),

in welcher

| | |
|---|---|
| E, $Z^1$, $Z^2$, $Z^3$ und $Z^4$ | die oben angegebenen Bedeutungen haben und |
| $Y^2$ | für Halogen, Alkylsulfonyloxy, Arylsulfonyloxy oder Alkanoyloxy steht, |

gegebenenfalls in Gegenwart eines Verdünnungsmittels und gegebenenfalls in Gegenwart eines Säureakzeptors, umsetzt.

Die zur Durchführung des erfindungsgemäßen Verfahrens o) als Ausgangsstoffe benötigten Benzofurandionmonooxime der Formel (XI) sind bereits bei der Beschreibung des erfindungsgemäßen Verfahrens g) beschrieben worden.

Die zur Durchführung des erfindungsgemäßen Verfahrens e) als Ausgangsstoffe benötigten O-Alkyl-benzofurandiondioxime sind durch die Formel (VIII) allgemein definiert. In dieser Formel (VIII) haben $R^1$, $R^2$, $R^3$ und $R^4$ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für $R^1$, $R^2$, $R^3$ und $R^4$ angegeben wurden.

Die Ausgangsstoffe der Formel (VIII) sind noch nicht bekannt und als neue Stoffe ebenfalls Gegenstand der vorliegenden Anmeldung.

Die O-Hydroxyethyl-benzofurandionmonooxime der Formel (VIII) werden erhalten, wenn man
Verfahren h) Benzofurandionmonooxime der Formel (XI) mit einem Alkoxyamin der Formel (IV),
gegebenenfalls in Gegenwart eines Verdünnungsmittels und gegebenenfalls in Gegenwart eines Säureakzeptors, umsetzt, oder wenn man
Verfahren p) Benzofurandiondioxime der Formel

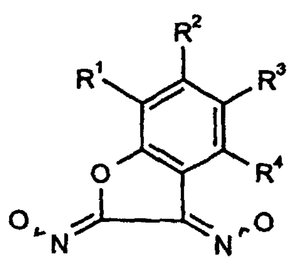

(XII)

in welcher

| | |
|---|---|
| $R^1$, $R^2$, $R^3$ und $R^4$ | die oben angegebenen Bedeutungen haben, |

mit einem Alkylierungsmittel der Formel (VI),
gegebenenfalls in Gegenwart eines Verdünnungsmittels, und gegebenenfalls in Gegenwart einer Base, umsetzt.

Die zur Durchführung des erfindungsgemäßen Verfahrens h) als Ausgangsstoffe benötigten Benzofurandionmonooxime der Formel (XI) sind bereits bei der Beschreibung des erfindungsgemäßen Verfahrens g) beschrieben worden.

Die zur Durchführung des erfindungsgemäßen Verfahrens p) als Ausgangsstoffe benötigten Benzofurandiondioxime sind durch die Formel (XII) allgemein definiert. In dieser Formel (XII) haben $R^1$, $R^2$, $R^3$ und $R^4$ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für $R^1$, $R^2$, $R^3$ und $R^4$ angegeben wurden.

Die Benzofurandiondioxime der Formel (XII) sind bekannt und können nach bekannten Verfahren hergestellt werden (vergleiche Chem. Ber. 42 (1909), 202).

Die zur Durchführung des erfindungsgemäßen Verfahrens f) als Ausgangsstoffe benötigten O-Hydroxyethylbenzofurandiondioxime sind durch die Formel (X) allgemein definiert. In dieser Formel (X) haben $R^1$, $R^2$, $R^3$ und $R^4$ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für $R^1$, $R^2$, $R^3$ und $R^4$ angegeben wurden.

Die Ausgangsstoffe der Formel (X) sind noch nicht bekannt und als neue Stoffe ebenfalls Gegenstand der vorliegenden Anmeldung.

Die O-Hydroxyethyl-benzofurandiondioxime der Formel (X) werden erhalten, wenn man

Verfahren i) O-Hydroxyethyl-benzofurandionmonooxime der Formel (VII),

mit Hydroxylamin - oder einem Säureadditionskomplex davon -

gegebenenfalls in Gegenwart eines Verdünnungsmittels und gegebenenfalls in Gegenwart eines Säureakzeptors, umsetzt, oder wenn man

Verfahren q) O-Oxyethyl-benzofurandiondioxime der Formel

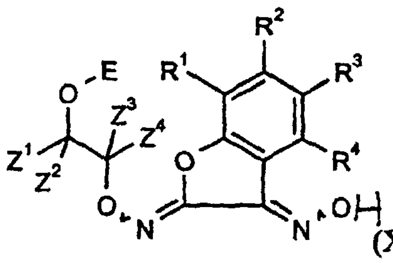

(XVI),

in welcher

E, $R^1$, $R^2$, $R^3$, $R^4$, $Z^1$, $Z^2$, $Z^3$ und $Z^4$ die oben angegebenen Bedeutungen haben,

mit Wasser oder einem Alkohol, gegebenenfalls in Gegenwart eines Verdünnungsmittels und gegebenenfalls in Gegenwart einer Säure oder einer Base, umsetzt.

Die zur Durchführung des erfindungsgemäßen Verfahrens i) als Ausgangsstoffe benötigten O-Hydroxyethylbenzofurandionmonooxime der Formel (VII) sind bereits bei der Beschreibung des erfindungsgemäßen Verfahrens d) beschrieben worden.

Die zur Durchführung des erfindungsgemäßen Verfahrens q) als Ausgangsstoffe benötigten O-Oxyethyl-benzofurandiondioxime sind durch die Formel (XVI) allgemein definiert. In dieser Formel (XVI) haben $R^1$, $R^2$, $R^3$, $R^4$, $Z^1$, $Z^2$, $Z^3$ und Z4 vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für $R^1$, $R^2$, $R^3$, $R^4$, $Z^1$, $Z^2$, $Z^3$ und $Z^4$ angegeben wurde. E hat vorzugsweise bzw. insbesondere diejenigen Bedeutungen, die weiter unten im Zusammenhang mit der Beschreibung der erfindungsgemäßen Verbindungen der Formel (XIII) als bevorzugt bzw. als insbesondere bevorzugt für E angegeben werden.

Die Ausgangsstoffe der Formel (XVI) sind noch nicht bekannt und als neue Stoffe ebenfalls Gegenstand der vorliegenden Anmeldung.

Die O-Oxyethyl-benzofurandiondioxime der Formel (XVI) werden erhalten, wenn man

Verfahren r) O-Oxyethyl-benzofurandionmonooxime der Formel (XIV) mit Hydroxylamin - oder einem Säureadditionskomplex davon -

gegebenenfalls in Gegenwart eines Verdünnungsmittels und gegebenenfalls in Gegenwart eines Säureakzeptors, umsetzt.

Die zur Durchführung des erfindungsgemäßen Verfahrens r) als Ausgangsstoffe benötigten O-Oxyethyl-benzofurandionmonooxime der Formel (XIV) sind bereits bei der Beschreibung des erfindungsgemäßen Verfahrens n) beschrieben worden.

Die zur Durchführung des erfindungsgemäßen Verfahrens m) als Ausgangsstoffe benötigten O-Oxyethyl-O'-methyl-benzofurandiondioxime der Formel (XIII), sind durch die Formel (III) allgemein definiert. In dieser Formel (XIII) haben A, $R^1$, $R^2$, $R^3$, $R^4$, $Z^1$, $Z^2$, $Z^3$ und $Z^4$ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für $R^1$, $R^2$, $R^3$, $R^4$, $Z^1$, $Z^2$, $Z^3$ und $Z^4$ angegeben wurden. E steht für eine Acylgruppe, vorzugsweise für Formyl, Acetyl oder Benzoyl, oder eine ketalische Schutzgruppe, vorzugsweise für 2-Tetrahydropyranyl, 1-Methoxy-1-ethyl, 1-Ethoxy-1-ethyl, Methoxymethyl oder Ethoxymethyl.

Die Ausgangsstoffe der Formel (XIII) sind noch nicht bekannt und als neue Stoffe ebenfalls Gegenstand der vorliegenden Anmeldung.

Die O-Oxyethyl-O'-methyl-benzofürandiondioxime der Formel (XIII) werden erhalten, wenn man
Verfahren s) O-Oxyethyl-benzofurandionmonooxime der Formel (XIV)
mit einem Alkoxyamin der Formel (IV)
- oder einem Säureadditionskomplex davon -
gegebenenfalls in Gegenwart eines Verdünnungsmittel und gegebenenfalls in Gegenwart eines Säureakzeptors, umsetzt, oder wenn man
Verfahren t) O-Oxyethyl-benzofurandiondioxime der Formel (XVI) mit einem Alkylierungsmittel der Formel (VI),

$$A - X \qquad (VI)$$

in welcher

A die in Anspruch 1 angegebene Bedeutung hat und
X für Halogen, Alkylsulfonyloxy, Alkoxysulfonyloxy oder Arylsulfonyloxy steht,

gegebenenfalls in Gegenwart eines Verdünnungsmittels, und gegebenenfalls in Gegenwart einer Base, umsetzt oder wenn man
Verfahren u) O-Alkyl-benzofurandiondioxime der Formel (VIII) mit einem Ethanolderivat der Formel (XV),
gegebenenfalls in Gegenwart eines Verdünnungsmittels und gegebenenfalls in Gegenwart eines Säureakzeptors, umsetzt.

Die zur Durchführung des erfindungsgemäßen Verfahrens s) als Ausgangsstoffe benötigten O-Oxyethyl-benzofurandionmonooxime der Formel (XIV) sind bereits bei der Beschreibung des erfindungsgemäßen Verfahrens n) beschrieben worden.

Die zur Durchführung des erfindungsgemäßen Verfahrens t) als Ausgangsstoffe benötigten O-Oxyethyl-benzofurandiondioxime der Formel (XVI) sind bereits bei der Beschreibung des erfindungsgemäßen Verfahrens q) beschrieben worden.

Die zur Durchführung des erfindungsgemäßen Verfahrens u) als Ausgangsstoffe benötigten O-Alkyl-benzofurandiondioxime der Formel (VIII) sind bereits bei der Beschreibung des erfindungsgemäßen Verfahrens e) beschrieben worden.

Die weiterhin zur Durchführung der erfindungsgemäßen Verfahren d), h) und s) als Ausgangsstoffe benötigten Alkoxyamine sind durch die Formel (IV) allgemein definiert. In dieser Formel (IV) hat A vorzugsweise bzw. insbesondere diejenige Bedeutung, die bereits im Zusammenhang mit der Beschreibung der erfindungsgemäß herstellbaren Verbindungen der Formel (I) als bevorzugt bzw. als insbesondere bevorzugt für A angegeben wurde. Bevorzugte Säureadditionskomplexe der Alkoxyamine der Formel (IV) sind deren Hydrochloride, Sulfate und Hydrogensulfate.

Die Alkoxyamine der Formel (IV) und deren Säureadditionskomplexe sind bekannte Synthesechemikalien.

Die weiterhin zur Durchführung der erfindungsgemäßen Verfahren f), p) und t) als Ausgangsstoffe benötigten Alkylierungsmittel sind durch die Formel (VI) allgemein definiert. In dieser Formel (VI) hat A vorzugsweise bzw. insbesondere diejenige Bedeutung, die bereits im Zusammenhang mit der Beschreibung der erfindungsgemäß herstellbaren Verbindungen der Formel (I) als bevorzugt bzw. als insbesondere bevorzugt für A angegeben wurde. X steht für Halogen, vorzugsweise Chlor, Brom oder Iod, Alkylsulfonyloxy, vorzugsweise Methylsulfonyloxy, Alkoxysulfonyloxy, vorzugsweise Methoxysulfonyloxy, oder Arylsulfonyloxy, vorzugsweise 4-Tolylsulfonyloxy.

Die Alkylierungsmittel der Formel (VI) sind bekannte Synthesechemikalien.

Die weiterhin zur Durchführung der erfindungsgemäßen Verfahren e) und g) als Ausgangsstoffe benötigten Ethanderivate sind durch die Formel (IX) allgemein definiert. In dieser Formel (IX) haben $Z^1$, $Z^2$, $Z^3$ und $Z^4$ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt fiir $Z^1$, $Z^2$, $Z^3$ und $Z^4$

angegeben wurden. $Y^1$ steht für Halogen, vorzugsweise Chlor, Brom oder Iod, Alkylsulfonyloxy, vorzugsweise Methylsulfonyloxy, Arylsulfonyloxy, vorzugsweise 4-Tolylsulfonyloxy, oder Alkanoyloxy, vorzugsweise Acetyloxy. G steht für Wasserstoff oder ist durch eine Einfachbindung mit $Y^1$ verbunden, wobei $Y^1$ fiir Sauerstoff steht und G für Carbonyl steht, oder G steht gemeinsam mit $Y^1$ auch für eine Einfachbindung.

Die Ethanderivate der Formel (IX), sind bekannte Synthesechemikalien.

Das weiterhin zur Durchführung der erfindungsgemäßen Verfahren i) und r) als Ausgangsstoff benötigte Hydroxylamin, oder seine Säureadditionskomplexe, vorzugsweise sein Hydrochlorid, Sulfat und Hydrogensulfat, sind bekannte Synthesechemikalien.

Die weiterhin zur Durchführung der erfindungsgemäßen Verfahren o) und u) als Ausgangsstoffe benötigten Ethanolderivate sind durch die Formel (XV) allgemein definiert. In dieser Formel (XV) haben $Z^1$, $Z^2$, $Z^3$ und $Z^4$ vorzugsweise bzw. insbesondere diejenigen Bedeutungen, die bereits im Zusammenhang mit der Beschreibung der erfindungsgemäß herstellbaren Verbindungen der Formel (I) als bevorzugt bzw. als insbesondere bevorzugt für $Z^1$, $Z^2$, $Z^3$ und $Z^4$ angegeben wurden. E hat vorzugsweise bzw. insbesondere diejenigen Bedeutung, die bereits im Zusammenhang mit der Beschreibung der erfindungsgemäßen Verbindungen der Formel (XIII) als bevorzugt bzw. als insbesondere bevorzugt für E angegeben wurde. $Y^2$ steht für Halogen, vorzugsweise Chlor, Brom oder Iod, Alkylsulfonyloxy, vorzugsweise Methylsulfonyloxy, Arylsulfonyloxy, vorzugsweise 4-Tolylsulfonyloxy, oder Alkanoyloxy, vorzugsweise Acetyloxy.

Die Ethanolderivate der Formel (XV) sind bekannt und können nach bekannten Methoden hergestellt werden (vergleiche z. B. Newkome, George R.; Marston, Charles R., J.Org.Chem., 50, 22, 1985, 4238-4245; Henry, Chem. Ber., 7 <1874>,70).

Wird das erfindungsgemäße Verfahren a) in Gegenwart einer Säure durchgeführt, kommen als Verdünnungsmittel alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Dioxan, 1,2- Dimethoxyethan, 1,2-Diethoxyethan oder Anisol; Ester wie Essigsäuremethylester oder Essigsäureethylester, oder Sulfone, wie Sulfolan sowie beliebige Mischungen der genannten Verdünnungsmittel. Besonders bevorzugte Verdünnungsmittel sind Ether, wie Diethylether, 1,2-Diethoxyethan oder Anisol; aromatische Kohlenwasserstoffe, wie beispielsweise Benzol, Toluol oder Xylol.

Wird das erfindungsgemäße Verfahren a) in Gegenwart einer Base durchgeführt, kommen als Verdünnungsmittel Wasser, sowie alle organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Nitrile, wie Acetonitril, Propionitril, n- oder i-Butyronitril oder Benzonitril; Amide, wie N, N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester, Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, sowie deren Gemische mit Wasser. Bevorzugte Verdünnungsmittel sind Wasser, Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether; sowie deren Gemische mit Wasser. Besonders bevorzugte Verdünnungsmittel sind in diesem Fall Wasser oder Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, sowie deren Gemische mit Wasser.

Als Verdünnungsmittel zur Durchführung der erfindungsgemäßen Verfahren d), h), i), r) und s) kommen alle inerten organischen Lösungsmittel in Betracht. Hierzu gehören vorzugsweise aromatische Kohlenwasserstoffe, wie beispielsweise Benzol, Toluol oder Xylol; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Amide, wie N,N-Dimethylformamid, N,N-Dimethylacetamid, N-Methylformanilid, N-Methylpyrrolidon oder Hexamethylphosphorsäuretriamid; organische Säuren, wie Essigsäure; Ester wie Essigsäuremethylester oder Essigsäure-ethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser. Besonders bevorzugte Verdünnungsmittel sind Amide, wie N,N-Dimethylformamid, N,N-Dimethylacetamid, N-Methylformanilid, N-Methylpyrrolidon oder Hexamethylphosphorsäuretriamid; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, Säuren, wie Essigsäure, deren Gemische mit Wasser oder reines Wasser. Weiterhin besonders bevorzugt sind auch Zweiphasengemische, wie beispielsweise Wasser/Toluol.

Als Verdünnungsmittel zur Durchführung der erfindungsgemäßen Verfahren e), f), g), o), p), t) und u) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser. Besonders bevorzugte Verdünnungsmittel sind Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether oder Diethylenglykolmonoethylether. Weiterhin besonders bevorzugt sind auch Zweiphasengemische, wie beispielsweise Wasser/Toluol.

Als Verdünnungsmittel zur Durchführung der erfindungsgemäßen Verfahren m), n) und q) kommen Wasser, sowie alle organischen Lösungsmittel in Betracht. Hierzu gehören vorzugsweise Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Das erfindungsgemäße Verfahren a) wird gegebenenfalls in Gegenwart einer Säure oder einer Base durchgeführt. Als Säuren kommen alle anorganischen und organischen Protonen- wie auch Lewissäuren, sowie auch alle polymeren Säuren infrage. Hierzu gehören beispielsweise Chlorwasserstoff, Bromwasserstoff, Schwefelsäure, Ameisensäure, Essigsäure, Trifluoressigsäure, Methansulfonsäure, Trifluormethansulfonsäure, Toluolsulfonsäure, Bortrifluorid (auch als Etherat), Bortribromid, Aluminiumtrichlorid, Zinkchlorid, Eisen-III-chlorid, Antimonpentachlorid, saure Ionenaustauscher, saure Tonerden und saures Kieselgel. Vorzugsweise kommen Chlorwasserstoff oder Bromwasserstoff infrage. Als Basen kommen alle üblichen anorganischen oder organischen Basen infrage. Hierzu gehören vorzugsweise Erdalkalimetalloder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU). Besonders bevorzugte Basen sind Natrium-methylat, Natrium-ethylat, Kalium-tert.butylat, Natriumhydroxid, Kaliumhydroxid, Ammoniumhydroxid, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die erfindungsgemäßen Verfahren d), h), i), r) und s) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydroxide, -alkoholate, -acetate, -carbonate oder -hydrogencarbonate, wie beispielsweise Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die erfindungsgemäßen Verfahren e), f), g), o), p), t) und u) werden gegebenenfalls in Gegenwart eines

geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Natriumcarbonat, Kaliumcarbonat, Kaliumhydrogencarbonat oder Natriumhydrogencarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens a) in einem größeren Bereich variiert werden. Im allgemeinen arbeitet man bei Temperaturen von -20°C bis 100°C, vorzugsweise bei Temperaturen von -10°C bis 80°C.

Die Reaktionstemperaturen können bei der Durchführung der erfindungsgemäßen Verfahren d), h), i), r und s) in einem größeren Bereich variiert werden. Im allgemeinen arbeitet man bei Temperaturen von 0°C bis 200°C, vorzugsweise bei Temperaturen von 20°C bis 150°C.

Die Reaktionstemperaturen können bei der Durchführung der erfindungsgemäßen Verfahren e), f), g), o), p), t) und u) in einem größeren Bereich variiert werden. Im allgemeinen arbeitet man bei Temperaturen von -20°C bis 100°C, vorzugsweise bei Temperaturen von 0°C bis 80°C.

Das erfindungsgemäße Verfahren a) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

In einer bevorzugten Verfahrensvariante (A) wird ein Benzofurandionmonooxim der Formel (XI) durch Reaktion mit einem Ethanderivat der Formel (IX) zunächst, wie in Verfahren g) beschrieben, in ein O-Hydroxyethyl-benzofurandionmonooxim der Formel (VII) überführt. Dieses setzt man ohne weitere Reinigung anschließend mit einem Alkoxyamin der Formel (IV) - oder einem Säureadditionskomplex davon - gegebenenfalls in einem Puffersystem, wie beispielsweise Natriumacetat/Essigsäure, wie in Verfahren d) beschrieben, zu einem O-Hydroxyethyl-O'-methyl-benzonfurandiondioxim der Formel (II) um, das wiederum ohne weitere Reinigung nach Behandlung mit einer Säure oder einer Base, gemäß Verfahren a), das gewünschte 3-(1-Hydroxyphenyl-1-alkoximinomethyl)dioxazin der Formel (I) liefert.

In einer dritten bevorzugten Verfahrensvariante (C) wird ein Benzofurandionmonooxim der Formel (XI) durch Reaktion mit einem Alkoxyamin der Formel (TV) - oder einem Säureadditionskomplex davon - zunächst in ein O-Alkylbenzofurandiondioxim der Formel (VIII) überführt. Dieses setzt man anschließend mit einem Ethanderivat der Formel (IX) zu einem O-Hydroxyethyl-O'-methyl-benzofurandiondioxim der Formel (II) um, das nach Behandlung mit einer Säure oder einer Base das gewünschte 3-(1-Hydroxyphenyl-l-alkoximinomethyl)dioxazin der Formel (I) liefert.

Es ist als äußerst überraschend zu bezeichnen, daß die erfindungsgemäßen Verfahren, insbesondere in ihrer Kombination, in hoher Ausbeute und hoher Reinheit ablaufen. In Chem. Ber. 1902, 1640 ist beispielsweise beschrieben, daß Benzofurandionmonooxime der Formel (XI) sowohl durch Behandlung mit Säuren, wie auch durch Behandlung mit Basen zu Salicylsäurederivaten oder Hydroxyphenylglyoxylsäurederivaten gespalten werden. Somit konnte nicht damit gerechnet werden, daß sich die 3-(l-Hydroxyphenyl-1-alkoximinomethyl)dioxazine in einer nur dreistufigen Reaktion ohne nennenswerte Nebenreaktionen herstellen lassen.

Die erfindungsgemäßen Verfahren weisen eine Reihe von Vorteilen auf. So ermöglichen sie die Herstellung einer Vielzahl von 3-(1-Hydroxyphenyl-1-alkoximinomethyl)dioxazinen in hoher Ausbeute und hoher Reinheit. Günstig ist es auch, daß die als Ausgangsstoffe benötigten Benzofurandionmonooxime in einfacher Weise auch in größeren Mengen zugänglich sind (Beilstein, E (II) 17, 462; Mameli, G. 56, 768).

**Verfahrens- und Herstellungsbeispiele:**

**Beispiel 1**

**Verfahrensvariante (A)**

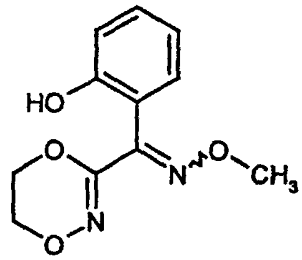

(1)

**1 Stufe**

Verbindung (VII-1):

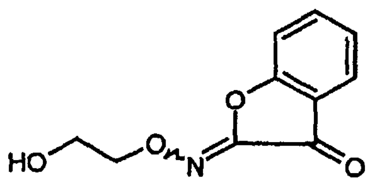

Verfahren g)
11,8 g (0,0725 Mol) Benzofuran-2,3-dion-2-oxim (XI-1) (Stoermer, Kahlert, B. 35, 1644) werden in 75 ml Dimethylformamid gelöst. Man gibt unter Kühlen 3 g (0,075 Mol) 60 %iges Natriumhydrid (Mineralölsuspension) portionsweise zu und rührt bei einer Temperatur von 25 °C ca. eine Stunde bis zum Ende der Gasentwicklung. Anschließend kühlt man auf 0 °C, tropft bei dieser Temperatur 9,3 g (0,0744 Mol) 2-Bromethanol zu und rührt noch 24 Stunden bei 25 °C. Man gießt das Reaktionsgemisch auf Wasser, extrahiert mit Essigsäureethylester, trocknet die organische Phase über Natriumsulfat und destilliert das Lösungsmittel im Vakuum ab. Der Rückstand wird aus einer Mischung von 150 ml Toluol und 100 ml Cyclohexan umkristallisiert. Man erhält 11,5 g (64 % der Theorie) Benzofuran-2,3-dion-2-[O-(2-hydroxy-ethyl)-oxim] (VII-1) (Gehalt nach HPLC-Analyse: 83,5 %). Eine aus Toluol umkristallisierte Probe schmilzt bei 110 - 111°C.

**2 Stufe**

Verbindung (II-1):

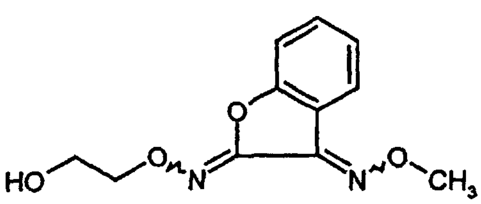

Verfahren d)
11,5 g rohes Benzofuran-2,3-dion-2-[O-(2-hydroxy-ethyl)-oxim] (VII-1) und 5,15 g (0,0616 Mol) O-Methylhydroxylamin-Hydrochlorid werden in 60 ml Dimethylformamid gelöst und 30 Minuten bei 80°C gerührt. Man gießt das Reaktionsgemisch auf Wasser und extrahiert mit Essigsäureethylester. Man trocknet die organische Phase über Natriumsulfat

und destilliert das Lösungsmittel im Vakuum ab. Man erhält 14,1 g eines Öles, das 40,5 % Benzofuran-2,3-dion-2-[O-(2-hydroxy-ethyl)-oxim]-3-(O-methyl-oxim) (II-1) und 14 % (5,6-Dihydro-[1,4,2]dioxazin-3-yl)-(2-hydroxy-phenyl)-methanon-O-methyl-oxim (1) enthält.

**3 Stufe**

Verbindung (1)

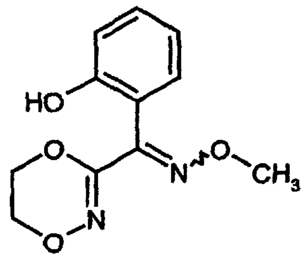

(1)

Verfahren a)

14,1 g des aus der 2. Stufe des Verfahrens A erhaltenen Öles, das die Verbindungen (II-1) und (1) enthält, werden in 200 ml Diethylether gelöst, welcher zuvor bei 0 °C mit Chlorwasserstoffgas gesättigt wurde. Man rührt 30 Minuten ohne weitere Kühlung und gießt die Mischung auf eine auf 0 °C gekühlte Natriumhydrogencarbonat-Lösung. Man trennt die organische Phase ab und extrahiert die wäßrige Phase noch mehrmals mit Diethylether. Die vereinigten organischen Phasen werden über Natriumsulfat getrocknet und bei vermindertem Druck eingeengt. Der Rückstand wird mit tert.-Butyl-methylether verrührt, wobei das Produkt kristallisiert. Man erhält 5,4 g (28% der Theorie, bezogen auf Benzofuran-2,3-dion-2-oxim) kristallines (5,6-Dihydro-[1,4,2]dioxazin-3-yl)-(2-hydroxy-phenyl)-methanon-O-methyl-oxim (1) (Gehalt nach HPLC-Analyse: 88,8 %).

$^1$H-NMR-Spektrum ($CDCl_3$/TMS): $\delta$ = 4,10 (3H); 4,19/4,20/4,21/4,22 (2H); 4,47/4,48/4,49/4,50 (2H); 6,26 (1H); 6,95-7,0 (2H); 7,21/7,23 (1H); 7,31-7,36 (2H) ppm.

**Beispiel 3:**

**Verfahrensvariante C**

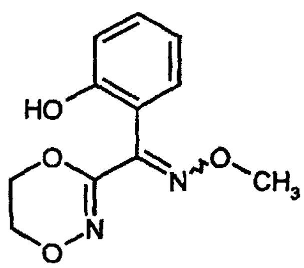

(1)

**1. Stufe**

Verbindung (VIII-1):

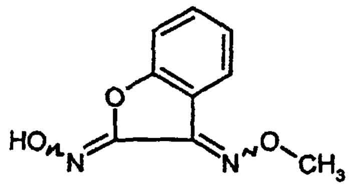

Verfahren h)

3,26 g (0,02 Mol) Benzofuran-2,3-dion-2-oxim (XI-1) werden in 20 ml Dimethylformamid mit 3,36 g (0,04 Mol) O-Methylhydroxylamin Hydrochlorid 20 Minuten bei 20°C und dann 45 Minuten bei 80°C gerührt. Man gießt in eine Mischung aus wäßriger Natriumhydrogencarbonat-Lösung und Essigsäureethylester. Man trennt die organische Phase ab, trocknet sie über Natriumsulfat und destilliert das Lösungsmittel bei vermindertem Druck ab. Der Rückstand wird mit Diethylether verrührt. Man erhält 1,4 g kristallines Benzofuran-2,3-dion-3-(O-methyl-oxim)-2-oxim (VIII-1), das nach HPLC-Analyse 70,7 % vom Stereoisomeren A und 8,5 % vom Stereoisomerem B enthält.

$^1$H-NMR-Spektrum ($CDCl_3$/TMS): $\delta$ = 4,09 (3H, Isomer B); 4,11 (3H, Isomer A); 7,21-7,35 (2H); 7,51-7,65 (1H, Isomer A und 2H, Isomer B); 8,02/8,04/8,05 (1H, Isomer A); 11,35 (1H, Isomer A); 11,74 (1H, Isomer B) ppm.

**2- Stufe**

Verbindung (II-1):

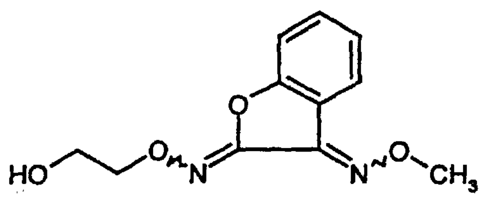

Verfahren e)

4,49 g (0,0234 Mol) Benzofuran-2,3-dion-3-(O-methyl-oxim)-2-oxim (VIII-1) werden in 25 ml Dimethylformamid gelöst. Zur Lösung gibt man 1 g (0,025 Mol) 60 %iges Natriumhydrid und rührt eine Stunde bei Raumtemperatur. Man gibt 3,1 g (0,0248 Mol) 2-Bromethanol zu und rührt 12 Stunden bei 25°C. Nach Zugabe von weiteren 0,5 g Natriummethylat und 1,22 g 2-Bromethanol rührt man 2 Stunden bei Raumtemperatur. Man gibt nochmals 0,5 g Natriummethylat und 1,22 g 2-Bromethanol zu und rührt weitere 2 Stunden bei Raumtemperatur. Man gießt das Reaktionsgemisch auf Wasser, extrahiert mit Essigsäureethylester, wäscht die organische Phase mit 3 mal 20 ml 2 N Natronlauge. Man destilliert das Lösungsmittel im Vakuum ab und chromatographiert den Rückstand in Diethylether/Petrolether (1:1) an Kieselgel. Man destilliert das Laufinittel im Vakuum ab und erhält 2,26 g (39,6% der Theorie) Benzofuran-2,3-dion-2-[O-(2-hydroxy-ethyl)-oxim]-3-(O-methyl-oxim) (II-1), das nach HPLC aus 84,29 % Stereoisomer A und aus 12,58 % Stereoisomer B besteht.

1H-NMR-Spektrum (CDCl3/TMS):

$\delta$ = 3,95-4,03 (2H); 4,20 (3H, Isomer B); 4,21 (3H, Isomer A); 4,37-4,40 (2H); 7,14-7,21 (2H); 7,40-7,49 (1H), 7,63/7,64/7,66 (1H, Isomer B); 8,04/8,06/8,07 (1H, Isomer A) ppm.

**3. Stufe**

Verbindung (1):

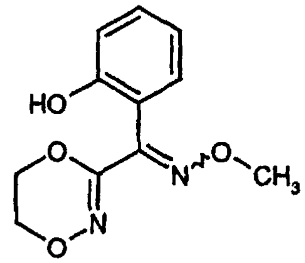

Verfahren a)

2 g Benzofuran-2,3-dion-2-[O-(2-hydroxy-ethyl)-oxim]-3-(O-methyl-oxim) (II-1) (Gehalt nach HPLC-Analyse 96,88 %, 0,0082 Mol), welches nach Verfahren e) hergestellt wurde, werden in 50 ml Diethylether gelöst, welcher zuvor bei 0 °C mit mit Chlorwasserstoffgas gesättigt wurde. Man rührt 30 Minuten ohne weitere Kühlung, destilliert anschließend das Lösungsmittel im Vakuum ab und versetzt den Rückstand wieder mit Diethylether. Ein Teil des Produktes kristallisiert aus und wird abfiltriert. Die Mutterlauge wird im Vakuum eingeengt und der Rückstand in 25 ml in bei 0 °C mit Chlorwasserstoffgas gesättigtem Diethylether gelöst. Man rührt wiederum 30 Minuten ohne weitere Kühlung, destilliert das Lösungsmittel im Vakuum ab und versetzt den Rückstand wiederum mit Diethylether. Es kristallisiert eine weiterer Teil des Produktes, der ebenfalls abfiltriert wird. Insgesamt erhält man 1,37 g (69 % der Theorie) (5,6-Dihydro-[1,4,2] dioxazin-3-yl)-(2-hydroxy-phenyl)-methanon-O-methyl-oxim (1) (Gehalt nach HPLC-Analyse: 97,59 %).

$^1$H-NMR-Spektrum ($CDCl_3$/TMS):

δ = 4,09 (3H); 4,18/4,19/4,20/4,21 (2H); 4,47/4,48/4,50 (2H); 6,94/6,95/6,97(2H); 7,20-7,36 (2H) ppm.

**Beispiel 4:**

**Verfahrensvariante (D)**

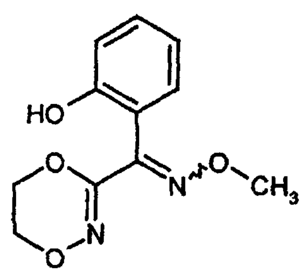

(1)

**1. Stufe:**

Verbindung (XIV-1):

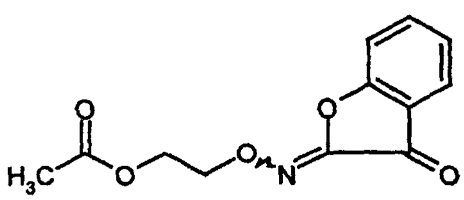

Verfahren o)

1,63 g (0,01 Mol) Benzofuran-2,3-dion-2-oxim (XI-1) werden in 5 ml N-Methyl-2-pyrrolidinon mit 1,14 g (0,0108 Mol) Natriumcarbonat 30 Minuten bei 20 °C gerührt. Nach Zugabe von 1,7 g (0,0102 Mol) Essigsäure-2-bromethylester rührt man 2 Stunden bei 70 °C. Man gießt die Mischung auf 30 ml Wasser und filtriert das Produkt ab. Man trocknet es und erhält *1,57* g (63 % der Theorie) kristallinen Essigsäure-2-(3-oxo-3H-benzofuran-2-ylidenaminooxy)-ethylester

(XIV-1).
$^1$H-NMR-Spektrum ($CDCl_3$/TMS): δ = 2,11 (3H); 4,43/4,44/4,45/4,46/4,47 (2H); 4,52/4,54/4,55/4,57 (2H); 7,28/7,30/7,33 (2H); 7,72/7,74/7,76/7,78/7,80 (2H) ppm.

**2. Stufe:**

Verbindung (XIII-1):

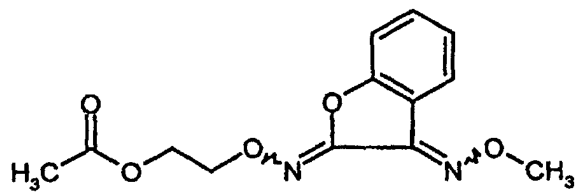

Verfahren s)
10 g (0,04 Mol) Essigsäure-2-(3-oxo-3H-benzofuran-2-ylidenaminooxy)-ethylester (XIV-1) und 4,18 g (0,05 Mol) O-Methylhydroxylamin-Hydrochlorid werden in 40 ml N-Methyl-2-pyrrolidinon gelöst und 1 Stunde bei 80°C gerührt. Man gießt das Reaktionsgemisch auf Wasser und extrahiert mit Essigsäureethylester. Man trocknet die organische Phase über Natriumsulfat und destilliert das Lösungsmittel im Vakuum ab. Man erhält 10,6 g Rohprodukt, welches mit tert. -Butylmethylether/Petrolether (1:1) an Kieselgel chromatographiert wird. Man erhält 6,9 g (59,1 % der Theorie) Essigsäure-2-(3-methoxyimino-3H-benzofuran-2-ylidenaminooxy)-ethylester (XIII-1) mit einem Gehalt von 82,15 % Isomer A und 13,38 % Isomer B (HPLC).
$^1$H-NMR-Spektrum ($CDCl_3$/TMS): δ = 2,10 (3H); 4,21 (3H, Isomer B); 4,22 (3H, Isomer A); 4,41-4,48 (4H); 7,15/7,17/7,20 (2H); 7,44-7,50 (1H); 7,63-7,66 (1H, Isomer B); 8,05-8,09 (1H, Isomer A) ppm.

**3. Stufe:**

Verbindung (II-1)

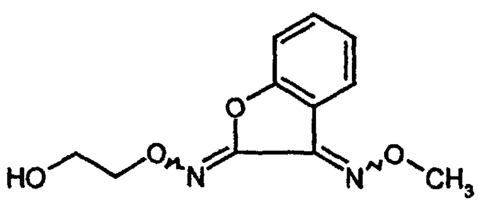

Verfahren m)
2 g (0,00718 Mol) Essigsäure-2-(3-methoxyimino-3H-benzofuran-2-ylidenaminooxy)-ethylester (XIII-1) werden in 8 ml Dimethylformamid bei 20°C mit 7,2 ml (0,0144 Mol) 2N Natronlauge versetzt und 16 Stunden bei 20°C gerührt. Man gießt das Reaktionsgemisch auf Wasser und extrahiert mit Essigsäureethylester. Man trocknet die organische Phase über Natriumsulfat und destilliert das Lösungsmittel im Vakuum ab. Man erhält 1,7 g (67,6 % der Theorie) Benzofuran-2,3-dion-2-[O-(2-hydroxy-ethyl)-oxim]-3-(O-methyl-oxim) (II-1), welches nach HPLC 56,8% des Stereoisomeren A und 10,7 % des Stereoisomeren B enthält.
Daten aus GC/MS-Analyse (die Substanz wurde vor der Analyse mit N-Methyl-N-trimethylsilyltrifluoracetamid silyliert):

Stereoisomer A:

Retentionsindex =2062

$M^+$ = 309, 308, 293, 249, 233, 192, 176, 145, 132, 89, 73, 45, 26.

Stereoisomer B:

Retentionsindex = 2000

$M^+$ = 309, 308, 293, 249, 218, 192, 176, 145, 132, 90, 73, 45.

**4. Stufe:**

Verbindung (1)

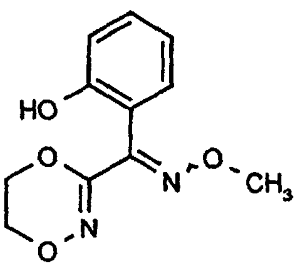

Diese Stufe ist als Verfahren a) in der 3. Stufe der Verfahrensvariante (A) und in der 3. Stufe der Verfahrensvariante (C) bereits beschrieben worden.

**Weitere Beispiele zu einzelnen Verfahren**

**Beispiel 5**

Verbindung (1):

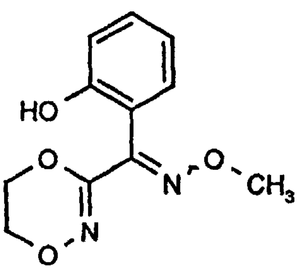

Verfahren c)
1,2 g (0,0054 Mol) Z-(5,6-Dihydro-[1,4,2]dioxazin-3-yl)-(2-hydroxy-phenyl)-methanon-oxim (V-1) werden in 5 ml Dimethylformamid mit 0,66 g (0,0062 Mol) Natriumcarbonat zunächst 30 Minuten bei 20 °C gerührt. Dann gibt man 0,83 g (0,00658 Mol) Dimethylsulfat zu und rührt weitere 16 Stunden bei 20 °C. Man stellt das Reaktionsgemisch mit 2N wäßriger Salzsäure schwach sauer und extrahiert mit Essigsäureethylester. Man trocknet die organische Phase über Natriumsulfat und destilliert das Lösungsmittel im Vakuum ab. Man erhält 0,8 g (50,35 % der Theorie) Z-(5,6-Dihydro-[1,4,2]dioxazin-3-yl)-(2-hydroxy-phenyl)-methanon-O-methyl-oxim (1) in einer Reinheit von 80,3 % (HPLC).
$^1$H-NMR-Spektrum ($CDCl_3$/TMS): $\delta$ = 4,08 (3H); 4,30/4,32/4,33 (2H); 4,53/4,54/4,55 (2H); 6,89/6,92/6,94/6,98/7,01 (2H); 7,28/7,31/7,33/7,34/7,35/7,37 (2H); 10,11 (1H) ppm.

**Beispiel 6**

Verbindung (II-1):

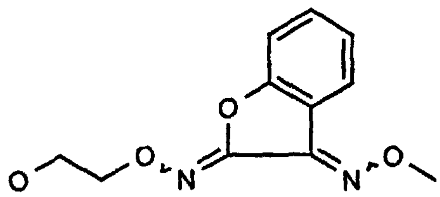

Verfahren e)

3,19 g (0,0166 Mol) Benzofuran-2,3-dion-3-(O-methyl-oxim)-2-oxim (VIII-1) (Gemisch aus 2 Stereoisomeren A:B = 13: 86) werden in 20 ml Methanol suspendiert und tropfenweise bei 20°C mit 8,3 g einer 2 molaren Natriummethylatlösung versetzt. Nachdem die Mischung homogen geworden ist, destilliert man das Methanol im Vakuum ab und trocknet den kristallinen Rückstand 12 Stunden im Exsiccator. Man erhält 3,55 g des Natriumsalzes von Benzofuran-2,3-dion-3-(O-methyl-oxim)-2-oxim (VIII-1). Dieses suspendiert man bei 20°C in 16 ml N-Methyl-2-pyrrolidinon und gibt 2,1 g (0,0168 Mol) 2-Bromethanol zu. Man rührt 48 Stunden bei 20 °C. Man gießt das Reaktionsgemisch auf Wasser, extrahiert mit Essigsäureethylester und wäscht die organische Phase mit 3 mal 20 ml 2 N Natronlauge. Man destilliert das Lösungsmittel im Vakuum ab und chromatographiert den Rückstand mit Diethylether/Petrolether (1:1) an Kieselgel. Man destilliert das Laufmittel im Vakuum ab und erhält 2,91 g (73,8 % der Theorie) Benzofuran-2,3-dion-2-[O-(2-hydroxy-ethyl)-oxim]-3-(O-methyl-oxim) (II-1), bestehend aus 88,6 % Stereoisomer B, 7,7 % Stereoisomer A und 3,2 % Stereoisomer C (HPLC).

$^1$H-NMR-Spektrum ($CDCl_3$/TMS): $\delta$ = 3,43 (1H, breit); 4,0-4,03 (2H); 4,20 (3H); 4,38-4,41 (2H); 7,14-7,21 (2H); 7,40/7,42/7,43/7,445 (1H); 7,63/7,64/7,66 (1H) ppm.

**Beispiel 7**

Verbindung (II-1):

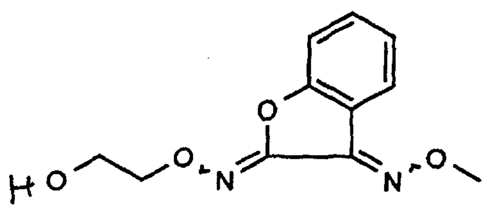

Verfahren f)

0,44 g (0,002 Mol) Benzofuran-2,3-dion-2-[O-(2-hydroxy-ethyl)-oxim]-3-oxim (X-1) werden bei 20°C in 5 ml Dimethylformamid 20 Minuten mit 0,08 g (0,002 Mol) 60 %igem Natriumhydrid gerührt. Nach beendeter Gasentwicklung gibt man 0,28 g (0,002 Mol) Methyljodid zu und rührt 16 Stunden bei 20 °C. Man gießt das Reaktionsgemisch auf Wasser, extrahiert mit Essigsäureethylester, trocknet die organische Phase über Natriumsulfat, destilliert das Lösungsmittel im Vakuum ab und erhält 0,5 g (35 % der Theorie) Rohprodukt. Dieses enthält gemäß der HPLC-Analyse 23 % des Stereoisomeren A und 10,1% des Stereoisomeren B von Benzofuran-2,3-dion-2-[O-(2-hydroxy-ethyl)-oxim]-3-(O-methyl-oxim) (II-1).

Daten aus GC/MS-Analyse (die Substanz wurde vor der Analyse mit N-Methyl-N-trimethylsilyltrifluoracetamid silyliert):

Retentionsindex = 2053 (Isomer A)
$M^+$ = 309, 308, 293, 249, 233, 192, 176, 145, 132, 89, 73, 45, 26.

Retentionsindex = 1997 (Isomer B)
$M^+$ = 309, 308, 293, 249, 218, 192, 176, 145, 132, 90, 73, 45.

Ferner enthält das Rohprodukt nach 20,5 % (HPLC) N-[2-(2-Hydroxy-ethoxyimino)-benzofuran-3-ylidene]-N-methylamin-N-oxid.
GC/MS-Analyse (silylierte Probe):
Retentionsindex = 2234

$M^+$ = 310, 308, 233, 192, 175, 159, 132, 102, 73, 45, 26).

**Beispiel 8**

Verbindung (X-1):

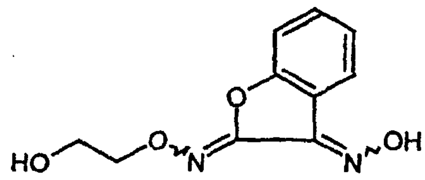

Verfahren i)
10,1 g (0,05 Mol) Benzofuran-2,3-dion-2-[O-(2-hydroxy-ethyl)-oxim] (VII-1) werden in 50 ml N-Methyl-2-pyrrolidinon mit 3,5 g Hydroxylamin Hydrochlorid 2 Stunden bei 80 °C gerührt. Man gießt das Reaktionsgemisch auf Wasser, extrahiert mit Essigsäure-ethylester, trocknet die organische Phase über Natriumsulfat und destilliert das Lösungsmittel im Vakuum ab. Dann chromatographiert man den Rückstand in tert.-Butyl-methylether/Petrolether (1:1) an Kieselgel. Man destilliert das Laufmittel im Vakuum ab und erhält 4,2 g (29,6 % der Theorie) Benzofuran-2,3-dion-2-[O-(2-hydroxy-ethyl)-oxim]-3-oxim, bestehend aus 62,4 % Isomer A und 15,8 % Isomer B (HPLC).
$^1$H-NMR-Spektrum (DMSO-$d_6$/TMS): δ = 3,64-3,71 (2H); 4,10-4,26 (2H); 4,78-4,87 (1H); 7,2-7,3 (1H); 7,3-7,4 (1H); 7-5-7,7 (1H); 8,11-8,14 (1H); 12,82 (1H, Isomer A); 12,91 (1H, Isomer B) ppm.

**Beispiel 10**

Verbindung (VII-1)

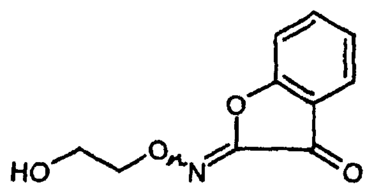

Verfahren n)
2,07 g (0,01 Mol) Benzofuran-2,3-dione-2-{O-[2-(tetrahydropyran-2-yloxy)-ethyl]-oxim} (XIV-2) werden in 12 ml Methanol gelöst und mit 100 mg saurem Ionenaustauscherharz 16 Stunden bei Raumtemperatur gerührt. Man versetzt das Reaktionsgemisch mit 40 ml Methanol, erwärmt bis die Kristalle gelöst sind und filtriert vom sauren Ionenaustauscherharz ab. Das Filtrat wird eingeengt und der Rückstand aus 10 ml Toluol umkristallisiert. Man erhält 1,69 g (81,5 % der Theorie) kristallines Benzofuran-2,3-dion-2-[O-(2-hydroxy-ethyl)-oxim] (VII-1) vom Schmelzpunkt 110-111 °C.
$^1$H-NMR-Spektrum ($CDCl_3$/TMS): δ = 2,33 (1H, breit), 4,00-4,03 (2H); 4,47-4,50 (2H); 7,27-7,32 (2H); 7,70-7,78 (2H) ppm.

**Beispiel 11**

Verbindung (XIV-2):

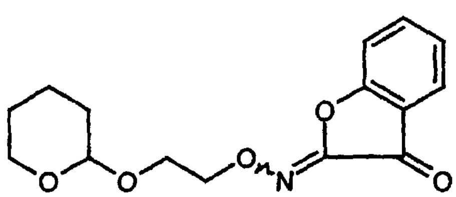

Verfahren o)
5 g (0,03 Mol) Benzofuran-2,3-dion-2-oxim (XI-1) werden in 30 ml Methanol gelöst und bei 20°C tropfenweise mit 15 ml 2 molarer Natriummethylatlösung in Methanol versetzt. Man destilliert das Lösungsmittel im Vakuum ab. Der kristalline Rückstand wird in 30 ml N-Methyl-2-pyrrolidinon gelöst und bei 20°C mit 6,27 g (0,03 Mol) 2-(2-Bromethoxy)-tetrahydropyran versetzt. Man rührt 16 Stunden bei 20°C, gießt das Reaktionsgemisch auf 100 ml Wasser und extrahiert zweimal mit je 100 ml Dichlormethan. Nach dem Abdestillieren des Lösungsmittels im Vakuum chromatographiert man den Rückstand in einer Mischung aus Diethylether/Dichlormethan/Petrolether (1:1:2) an Kieselgel. Man erhält 5,55 g (62,1% der Theorie) Benzofuran-2,3-dion-2-{O-[2-(tetrahydropyran-2-yloxy)-ethyl]-oxim} (XIV-2).
$^1$H-NMR-Spektrum ($CDCl_3$/TMS): δ = 1,50-1,86 (6H); 3,49-3,53 (1H); 3,81-3,88 (2H); 4,02-4,09 (1H); 4,52-4,55 (2H); 4,66-4,69 (1H); 7,26-7,31 (2H); 7,69-7,80 (2H) ppm.

**Beispiel 12**

Verbindung (VIII-1)

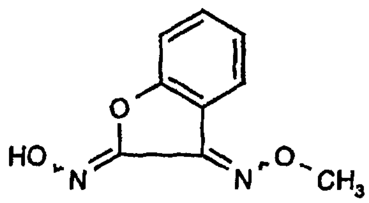

Verfahren p)
Zu einer Suspension von 2,4 g (0,06 Mol) 60%igem Natriumhydrid in 25 ml Dimethylformamid tropft man eine Lösung von 11 g (0,0617 Mol) Benzofurandion-2,3-dioxim in 50 ml Dimethylformamid und rührt eine Stunde bei 20°C. Dann tropft man 7,55 g (0,06 Mol) Dimethylsulfat zu und rührt weitere 16 Stunden bei 20°C. Man gießt das Reaktionsgemisch auf Wasser und extrahiert mit Essigsäureethylester, trocknet die organische Phase über Natriumsulfat und engt im Vakuum ein. Der Rückstand wird mit Hexan/Aceton (7:3) an Kieselgel chromatografiert. Nach dem Verrühren des Rückstandes mit Diethylether erhält man 0,7 g Benzofuran-2,3-dion-3-(O-methyl-oxim)-2-oxim (VIII-1) als Stereoisomerengemisch, bestehend aus 90,6 % Isomer A und 9 % Isomer B (HPLC).

**Beispiel 13**

Verbindung (X-1):

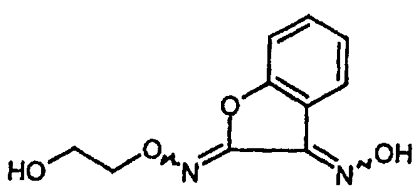

Verfahren q)
5,28 g (0,02 Mol) Essigsäure-2-(3-hydroxyimino-3H-benzofuran-2-ylideneaminooxy)-ethylester (XVI-1) werden in 20 ml Dimethylformamid mit 4,24 g (0,048 Mol) 45 %iger Natronlauge 3 Stunden bei 20 °C gerührt. Man säuert mit 2N Salzsäure an, extrahiert mit Essigsäureethylester, trocknet die organische Phase über Natriumsulfat und destilliert das Lösungsmittel im Vakuum ab. Das Rohprodukt enthält nach HPLC-Analyse 23,4 % Stereoisomer A und 4,1% des Stereoisomer B von Benzofuran-2,3-dion-2-[O-(2-hydroxy-ethyl)-oxim]-3-oxim (X-1). Nach dem Verrühren mit Diethylether erhält man 1,4 g (28,5 % der Theorie) kristallines Benzofuran-2,3-dion-2-[O-(2-hydroxy-ethyl)-oxim]-3-oxim (X-1), bestehend aus 84,7 % Stereoisomer A und 6 % Stereoisomer B (HPLC).
$^1$H-NMR-Spektrum (DMSO-$d_6$/TMS): δ = 3,64-3,71 (2H); 4,10-4,26 (2H); 4,78-4,87 (1H); 7,2-7,3 (1H); 7,3-7,4 (1H); 7-5-7,7 (1H); 8,11-8,14 (1H); 12,82 (1H, Stereoisomer A); 12,91 (1H, Stereoisomer B) ppm.

**Beispiel 14**

Verbindung (XVI-1):

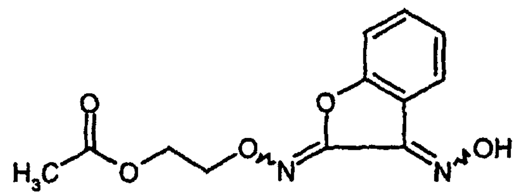

Verfahren r)
5 g (0,02 Mol) Essigsäure-2-(3-oxo-3H-benzofuran-2-ylidenaminooxy)-ethylester (XIV-1) und 1,74 g (0,025 Mol) Hydroxylamin-Hydrochlorid werden in 20 ml Dimethylformamid gelöst und 2 Stunden bei 100°C gerührt. Man gießt das Reaktionsgemisch auf Wasser und extrahiert mit Essigsäureethylester. Man trocknet die organische Phase über Natriumsulfat und destilliert das Lösungsmittel im Vakuum ab. Der Rückstand wird mit tert.-Butylmethylether/Petrolether (1:1) an Kieselgel chromatographiert. Man erhält 2,7 g (38,5 % der Theorie) Essigsäure-2-(3-hydroxyimino-3H-benzofuran-2-ylideneaminooxy)-ethylester (XVI-1), bestehend aus 64,23 % Stereoisomer A und 14,9 % Stereoisomer B (HPLC).
$^1$H-NMR-Spektrum ($CDCl_3$/TMS): $\delta$ = 2,11 (3H, Stereoisomer A); 2,12 (3H, Stereoisomer B); 4,43-4,46 (4H); 7,19-7,23 (2H); 7,46-7,52 (1H); 7,65-7,8 (1H, Stereoisomer B); 8,16/8,17/8,19 (1H, Stereoisomer A); 8,9 (1H) ppm.

**Beispiel 15**

Verbindung (XIII-1)

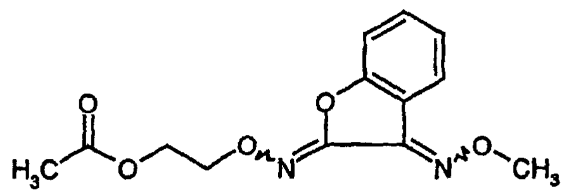

Verfahren s)
10 g (0,04 Mol) Essigsäure-2-(3-oxo-3H-benzofuran-2-ylidenaminooxy)-ethylester (XIV-1) und 4,18 g (0,05 Mol) O-Methylhydroxylamin-Hydrochlorid werden in 40 ml N-Methyl-2-pyrrolidinon gelöst und 1 Stunde bei 80°C gerührt. Man gießt das Reaktionsgemisch auf Wasser und extrahiert mit Essigsäureethylester. Man trocknet die organische Phase über Natriumsulfat und destilliert das Lösungsmittel im Vakuum ab. Man erhält 10,6 g rohen Essigsäure-2-(3-methoxyimino-3H-benzofuran-2-ylidenaminooxy)-ethylester (XIII-1), welcher mit einer Mischung aus tert.-Butylmethylether und Petrolether (1:1) an Kieselgel chromatographiert wird. Man erhält 6,9 g (59,1 % der Theorie) Essigsäure-2-(3-methoxyimino-3H-benzofuran-2-ylidenaminooxy)-ethylester (XIII-1) mit einem Gehalt von 82,15 % Stereoisomer A und 13,38 % Stereoisomer B.
$^1$H-NMR-Spektrum ($CDCl_3$/TMS): $\delta$ = 2,10 (3H); 4,21(3H, Stereoisomer B); 4,22(3H, Stereoisomer A); 4,41-4,48 (4H); 7,15/7,17/7,20 (2H); 7,44-7,50 (1H); 8,05-8,09 (1H) ppm.

**Beispiel 16**

Verbindung (XIII-1):

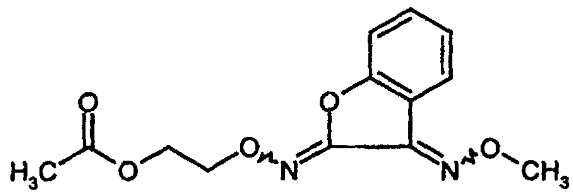

Verfahren t)
0,26 g (0,001 Mol) Essigsäure-2-(3-hydroxyimino-3H-benzofuran-2-ylidenaminooxy)-ethylester (XVI-1) werden in 2 ml Dimethylformamid gelöst. Man gibt 0,04 g (0,001 Mol) 60 %iges Natriumhydrid bei 20 °C dazu und rührt bis zum Ende der Gasentwicklung. Dann gibt man 0,05 g (0,0005 Mol) Natriumcarbonat und 0,13 g (0,001 Mol) Dimethylsulfat zum Reaktionsgemisch und läßt es 2 Tage bei 20 °C stehen. Man gießt das Reaktionsgemisch auf Wasser und extrahiert mit Essigsäure-ethylester. Man trocknet die organische Phase über Natriumsulfat und destilliert das Lösungsmittel im Vakuum ab. Man erhält 0,24 g (42,5 % der Theorie) rohen Essigsäure-2-(3-methoxyimino-3H-benzofuran-2-ylidenaminooxy)-ethylester (XIII-1), der nach HPLC-Analyse 33,9 % Stereoisomer A und 15,4 % Stereoisomer B enthält.
Daten aus der GC/MS-Analyse:

Retentionsindex = 2097 (Stereoisomer A)
$M^+$ = 279, 278, 218, 187, 160, 144, 130, 87, 75, 43, 26.

Retentionsindex = 2036 (Stereoisomer B)
$M^+$ = 279, 278, 218, 187, 160, 144, 130, 87, 63, 43, 26.

**Beispiel 17**

Verbindung (XIII-1)

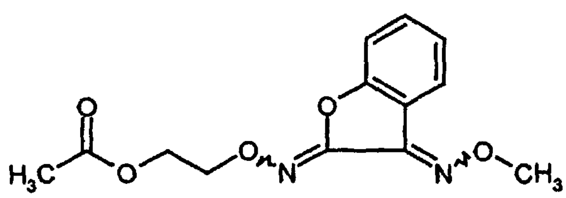

Verfahren u)
1,92 g (0,01 Mol) Benzofuran-2,3-dion-3-(O-methyl-oxim)-2-oxim (VIII-1) werden in 10 ml Dimethylformamid bei 20°C gelöst. Zu dieser Lösung gibt man 0,4 g (0,01 Mol) 60 %iges Natriumhydrid und rührt 1 Stunde bei 20°C. Nun gibt man 1,67 g (0,01 Mol) Essigsäure-2-bromethylester zu und rührt 16 Stunden bei 20 °C. Das Reaktionsgemisch wird auf Wasser gegossen und mit Essigsäureethylester extrahiert. Die organische Phase wird über Natriumsulfat getrocknet und im Vakuum eingeengt. Man erhält 2,3 g (52,6 % der Theorie) Essigsäure-2-(3-methoxyimino-3H-benzofuran-2-ylidenaminooxy)-ethylester (XIII-1), bestehend aus 51,17 % Stereoisomer A und 12,49 % Stereoisomer B (HPLC).
Daten aus GC/MS-Analyse (die Substanz wurde vor der Analyse mit N-Methyl-N-trimethylsilyltrifluoracetamid silyliert):

Retentionsindex = 2097 (Stereoisomer A)
$M^+$ = 279, 278, 218, 187, 160, 144, 130, 87, 75, 43, 26.

Retentionsindex = 2035 (Stereoisomer B)
$M^+$ = 279, 278, 218, 187, 160, 144, 130, 87, 75, 43, 26.

**Patentansprüche**

1. Verfahren zur Herstellung von Verbindungen der Formel (I)

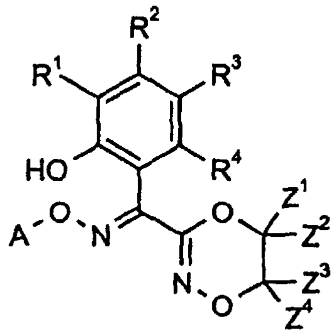

(I),

in welcher

| | |
|---|---|
| A | fiir Alkyl steht, |
| $R^1$, $R^2$, $R^3$ und $R^4$ | gleich oder verschieden sind und unabhängig voneinander jeweils für Wasserstoff, Halogen, Cyano, Nitro, jeweils gegebenenfalls durch Halogen substituiertes Alkyl, Alkoxy, Alkylthio, Alkylsulfinyl oder Alkylsulfonyl stehen und |
| $Z^1$, $Z^2$, $Z^3$ und $Z^4$ | gleich oder verschieden sind und unabhängig voneinander für Wasserstoff, Alkyl, Halogenalkyl oder Hydroxyalkyl stehen, oder |
| $Z^1$ und $Z^2$ oder $Z^1$ und $Z^3$ oder $Z^3$ und $Z^4$ | gemeinsam mit den jeweiligen Kohlenstoffatomen, an die sie gebunden sind, einen cycloaliphatischen Ring bilden, |

**dadurch gekennzeichnet, daß**

a) O-Hydroxyethyl-O'-methyl-benzofurandiondioxime der Formel

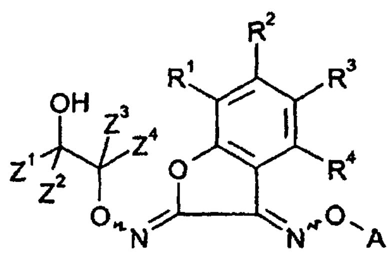

(II),

in welcher

| | |
|---|---|
| A, $R^1$, $R^2$, $R^3$, $R^4$, $Z^1$, $Z^2$, $Z^3$ und $Z^4$ | die oben angegebenen Bedeutungen haben, |

gegebenenfalls in Gegenwart eines Verdünnungsmittels und gegebenenfalls in Gegenwart einer Säure oder einer Base, umlagert.

**2.** Verfahren gemäß Anspruch 1, wobei in der Formel (I)

| | |
|---|---|
| A | fiir Methyl, Ethyl, n-, oder i-Propyl steht, |
| $R^1$, $R^2$, $R^3$ und $R^4$, | gleich oder verschieden sind und unabhängig voneinander jeweils für Wasserstoff, Halogen, Cyano, Nitro, jeweils gegebenenfalls durch 1 bis 5 Halogenatome substituiertes Alkyl, Alkoxy, Alkylthio, Alkylsulfi- |

nyl oder Alkylsulfonyl mit jeweils 1 bis 6 Kohlenstoffatomen, steht,

$Z^1$, $Z^2$, $Z^3$ und $Z^4$ gleich oder verschieden sind und unabhängig voneinander fiir Wasserstoff, Alkyl oder Hydroxyalkyl mit 1 bis 4 Kohlenstoffatomen oder Halogenalkyl mit 1 bis 4 Kohlenstoffatomen und 1 bis 5 gleichen oder verschiedenen Halogenatomen stehen, oder

$Z^1$ und $Z^2$ oder $Z^1$ und $Z^3$ oder $Z^3$ und $Z^4$ gemeinsam mit den jeweiligen Kohlenstoffatomen, an die sie gebunden sind, einen cyclo-aliphatischen Ring mit fünf, sechs oder sieben Kohlenstoffatomen bilden.

**3.** Verfahren gemäß Anspruch 1, wobei in der Formel (I)

A für Methyl oder Ethyl steht,

$R^1$, $R^2$, $R^3$ und $R^4$ gleich oder verschieden sind und unabhängig voneinander jeweils für Wasserstoff, Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl stehen, und

$Z^1$, $Z^2$, $Z^3$ und $Z^4$ gleich oder verschieden sind und unabhängig voneinander für Wasserstoff, Methyl, Ethyl, n- oder i-Propyl, n-, i-, s- oder t-Butyl, Hydroxymethyl, Trifluormethyl oder Trifluorethyl stehen, oder

$Z^1$ und $Z^2$ oder $Z^1$ und $Z^3$ oder $Z^3$ und $Z^4$ gemeinsam mit den jeweiligen Kohlenstoffatomen, an die sie gebunden sind, einen cycloaliphatischen Ring mit fünf, sechs oder sieben Kohlenstoffatomen bilden.

**4.** Verbindungen der Formel (II)

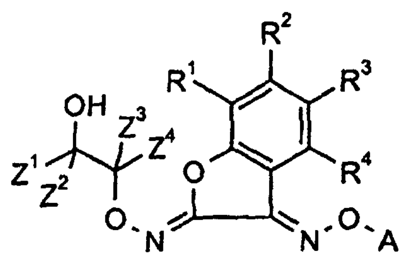

(II),

in welcher

A, $R^1$, $R^2$, $R^3$, $R^4$, $Z^1$, $Z^2$, $Z^3$ und $Z^4$ die in Anspruch 1 angegebenen Bedeutungen haben.

**5.** Verfahren zur Herstellung von Verbindungen der Formel (II) wie in Anspruch 4 definiert, **dadurch gekennzeichnet, daß** man

d) O-Hydroxyethyl-benzofurandionmonooxime der Formel

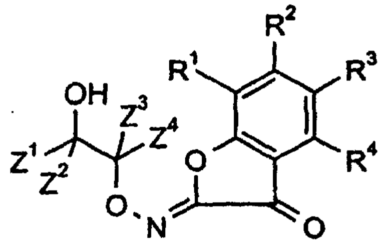

(VII),

in welcher

$R^1$, $R^2$, $R^3$, $R^4$, $Z^1$, $Z^2$, $Z^3$ und $Z^4$ die in Anspruch 1 angegebenen Bedeutungen haben,

mit einem Alkoxyamin der Formel (IV)

$$A - O - NH_2 \qquad (IV),$$

in welcher

A die in Anspruch 1 angegebene Bedeutung hat

- oder einem Säureadditionskomplex davon -
gegebenenfalls in Gegenwart eines Verdünnungsmittels und gegebenenfalls in Gegenwart eines Säureakzeptors, umsetzt, oder dass man

e) O-Alkyl-benzofurandiondioxime der Formel

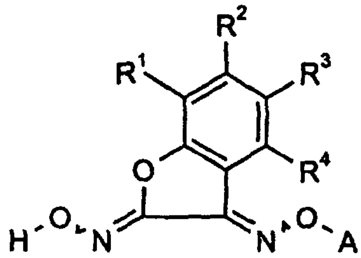

(VIII),

in welcher

A, $R^1$, $R^2$, $R^3$ und $R^4$ die in Anspruch 1 angegebenen Bedeutungen haben,

mit einem Ethanderivat der Formel

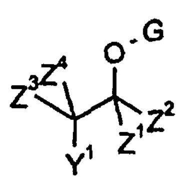

(IX),

in welcher

$Y^1$ fiir Halogen, Alkylsulfonyloxy, Arylsulfonyloxy oder Alkanoyloxy steht und

G für Wasserstoff steht, oder

$Y^1$ und G durch eine Einfachbindung miteinander verbunden sind, wobei

$Y^1$ für Sauerstoff steht oder

G für

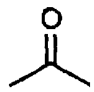

' steht, oder

$Y^1$ und G gemeinsam fiir eine Einfachbindung stehen und

$Z^1$, $Z^2$, $Z^3$ und $Z^4$ die in Anspruch 1 angegebenen Bedeutungen haben,

gegebenenfalls in Gegenwart eines Verdünnungsmittel, und gegebenenfalls in Gegenwart einer Base, umsetzt, oder daß man

f) O-Hydroxyethyl-benzofurandiondioxime der Formel

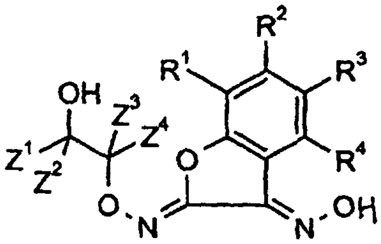

(X),

in welcher

$R^1$, $R^2$, $R^3$, $R^4$, $Z^1$, $Z^2$, $Z^3$ und $Z^4$ die in Anspruch 1 angegebenen Bedeutungen haben,

mit einem Alkylierungsmittel der Formel (VI),

$$A\text{-}X \qquad (VI),$$

in welcher A die in Anspruch 1 angegebene Bedeutung hat und X für Halogen, Alkylsulfonyloxy, Alkoxysulfonyloxy oder Arylsulfonyloxy steht
gegebenenfalls in Gegenwart eines Verdünnungsmittels, und gegebenenfalls in Gegenwart einer Base, umsetzt oder dass man

m) O-Oxyethyl-O'-methyl-benzofurandiondioxime der Formel

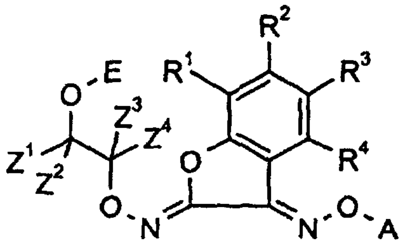

(XIII),

in welcher

A, $R^1$, $R^2$, $R^3$, $R^4$, $Z^1$, $Z^2$, $Z^3$ und $Z^4$ die in Anspruch 1 angegebenen Bedeutungen haben und

E für eine Acylgruppe oder eine ketalische Schutzgruppe steht,

mit Wasser oder einem Alkohol, gegebenenfalls in Gegenwart eines Verdünnungsmittels und gegebenenfalls in Gegenwart einer Säure oder einer Base, umsetzt.

**6.** Verbindungen der Formel (VII)

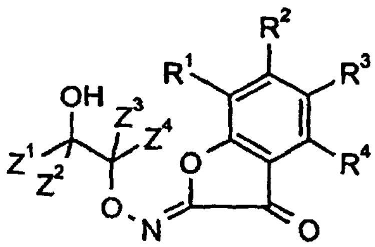

(VII),

in welcher

$R^1$, $R^2$, $R^3$, $R^4$, $Z^1$, $Z^2$, $Z^3$ und $Z^4$ die in Anspruch 1 angegebenen Bedeutungen haben.

**7.** Verfahren zur Herstellung von Verbindungen der Formel (VII) wie in Anspruch 6 definiert, **dadurch gekennzeichnet, daß** man

g) Benzofurandionmonooxime der Formel

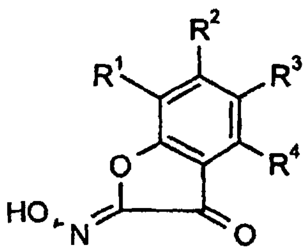

(XI),

in welcher

$R^1$, $R^2$, $R^3$ und $R^4$ die in Anspruch 1 angegebenen Bedeutungen haben,

mit einem Ethanderivat der Formel (IX),
gegebenenfalls in Gegenwart eines Verdünnungsmittels, und gegebenenfalls in Gegenwart einer Base, umsetzt, oder daß man

n) O-Oxyethyl-benzofurandionmonooxime der Formel

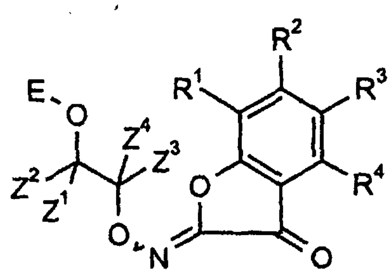

(XIV),

in welcher

$R^1$, $R^2$, $R^3$, $R^4$, $Z^1$, $Z^2$, $Z^3$ und $Z^4$ die in Anspruch 1 angegebenen Bedeutungen haben, und

E die in Anspruch 5 angegebenen Bedeutungen hat,

mit Wasser oder einem Alkohol, gegebenenfalls in Gegenwart eines Verdünnungsmittels und gegebenenfalls in Gegenwart einer Säure oder einer Base, umsetzt.

**8.** Verbindungen der Formel (XIV)

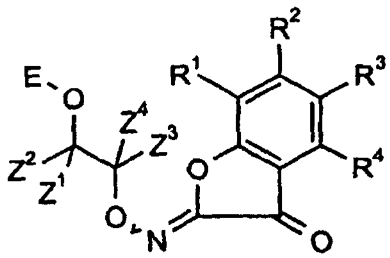

(XIV),

in welcher

$R^1$, $R^2$, $R^3$, $R^4$, $Z^1$, $Z^2$, $Z^3$ und $Z^4$ die in Anspruch 1 angegebenen Bedeutungen haben und

E für eine Acylgruppe oder eine ketalische Schutzgruppe steht.

**9.** Verfahren zur Herstellung von Verbindungen der Formel (XIV) wie in Anspruch 8 definiert, **dadurch gekennzeichnet, daß** man

o) Benzofurandionmonooxime der Formel (XI) mit einem Ethanolderivat der Formel

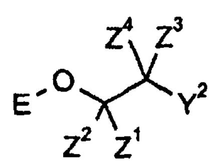

(XV),

in welcher

E, $Z^1$, $Z^2$, $Z^3$ und $Z^4$ die in Anspruch 8 angegebenen Bedeutungen haben und

$Y^2$ für Halogen, Alkylsulfonyloxy, Arylsulfonyloxy oder Alkanoyloxy steht,

gegebenenfalls in Gegenwart eines Verdünnungsmittels und gegebenenfalls in Gegenwart eines Säureakzeptors, umsetzt.

**10.** Verbindungen der Formel (X)

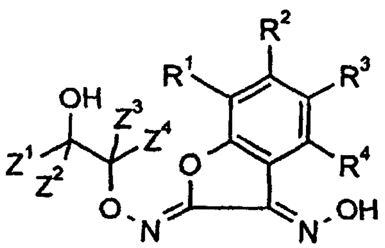

(X),

in welcher

$R^1$, $R^2$, $R^3$, $R^4$, $Z^1$, $Z^2$, $Z^3$ und $Z^4$ die in Anspruch 1 angegebenen Bedeutungen haben.

**11.** Verfahren zur Herstellung von Verbindungen der Formel (X) wie in Anspruch 10 definiert, **dadurch gekennzeichnet, daß** man

i) O-Hydroxyethyl-benzofurandionmonooxime der Formel (VII),
mit Hydroxylamin - oder einem Säureadditionskomplex davon -
gegebenenfalls in Gegenwart eines Verdünnungsmittels und gegebenenfalls in Gegenwart eines Säureakzeptors, umsetzt, oder daß man

q) O-Oxyethyl-benzofurandiondioxime der Formel

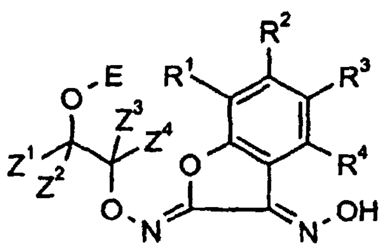

(XVI),

in welcher

E, $R^1$, $R^2$, $R^3$, $R^4$, $Z^1$, $Z^2$, $Z^3$ und $Z^4$ die in Anspruch 10 angegebenen Bedeutungen haben,

mit Wasser oder einem Alkohol, gegebenenfalls in Gegenwart eines Verdünnungsmittels und gegebenenfalls in Gegenwart einer Säure oder einer Base, umsetzt.

**12.** Verbindungen der Formel (XVI)

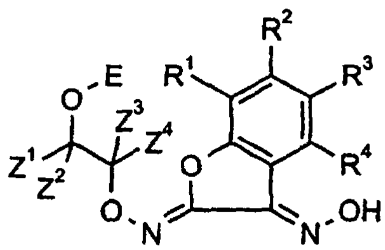

(XVI),

in welcher

$R^1$, $R^2$, $R^3$, $R^4$, $Z^1$, $Z^2$, $Z^3$ und $Z^4$ die in Anspruch 1 angegebenen Bedeutungen haben und

E für eine Acylgruppe oder eine ketalische Schutzgruppe steht.

**13.** Verfahren zur Herstellung von Verbindungen der Formel (XVI) wie in Anspruch 12 definiert, **dadurch gekennzeichnet, daß** man

r) O-Oxyethyl-benzofurandionmonooxime der Formel (XIV), wie in Anspruch 7 definiert
mit Hydroxylamin - oder einem Säureadditionskomplex davon -
gegebenenfalls in Gegenwart eines Verdünnungsmittels und gegebenenfalls in Gegenwart eines Säureakzeptors, umsetzt.

**14.** Verbindungen der Formel (XIII)

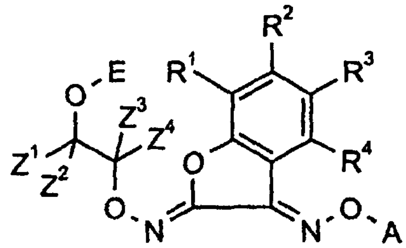

(XIII),

in welcher

A, $R^1$, $R^2$, $R^3$, $R^4$, $Z^1$, $Z^2$, $Z^3$ und $Z^4$ die in Anspruch 1 angegebenen Bedeutungen haben und

E für eine Acylgruppe oder eine ketalische Schutzgruppe steht.

**15.** Verfahren zur Herstellung von Verbindungen der Formel (XIII) wie in Anspruch 14 definiert, **dadurch gekennzeichnet, daß** man

s) O-Oxyethyl-benzofurandionmonooxime der Formel (XIV) wie in Anspruch 7 definiert, mit einem Alkoxyamin der Formel (IV) wie in Anspruch 5 definiert,
- oder einem Säureadditionskomplex davon -
gegebenenfalls in Gegenwart eines Verdünnungsmittel und gegebenenfalls in Gegenwart eines Säureakzeptors, umsetzt, oder daß man

t) O-Oxyethyl-benzofurandiondioxime der Formel (XVI) wie in Anspruch 12 definiert, mit einem Alkylierungsmittel der Formel (VI),

A - X (VI)

in welcher

A die in Anspruch 1 angegebene Bedeutung hat und

X für Halogen, Alkylsulfonyloxy, Alkoxysulfonyloxy oder Arylsulfonyloxy steht,

gegebenenfalls in Gegenwart eines Verdünnungsmittels, und gegebenenfalls in Gegenwart einer Base, umsetzt oder daß man

u) O-Alkyl-benzofurandiondioxime der Formel (VIII) wie in Anspruch 5 definiert, mit einem Ethanolderivat der Formel (XV) wie in Anspruch 9 definiert,
gegebenenfalls in Gegenwart eines Verdünnungsmittels und gegebenenfalls in Gegenwart eines Säureakzeptors, umsetzt.

**16.** Verwendung der Verbindungen der Formeln (II), (VII), (XIV), (X), (XVI) und (XIII) wie in den Ansprüchen 1, 4, 7, 10, 12 und 14 definiert, als Zwischenprodukte bzw. Vorprodukte zur Herstellung von Verbindungen mit fungiziden Eigenschaften.

**17.** Verbindung der Formel (II-1)

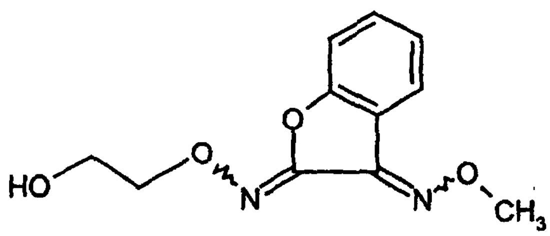

(II-1)

## Claims

**1.** Process for preparing compounds of the formula (I)

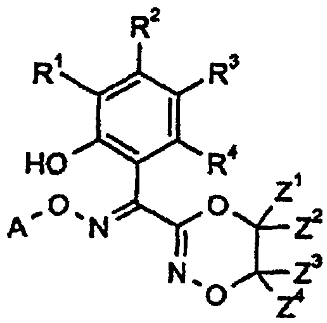

(I),

in which

A represents alkyl,

$R^1$, $R^2$, $R^3$ and $R^4$ are identical or different and each represent independently of one another hydrogen, halogen, cyano, nitro, respectively optionally halogen-substituted alkyl, alkoxy, alkylthio, alkylsulphinyl or alkylsulphonyl, and

$Z^1$, $Z^2$, $Z^3$ and $Z^4$ are identical or different and each represent independently of one another hydrogen, alkyl, halogenoalkyl or hydroxyalkyl, or

$Z^1$ and $Z^2$ or $Z^1$ and $Z^3$ or $Z^3$ and $Z^4$ form together with the respective carbon atoms that they are attached to a cycloaliphatic ring,

**characterized in that**

a) O-hydroxyethyl-O'-methyl-benzofurandione dioximes of the formula

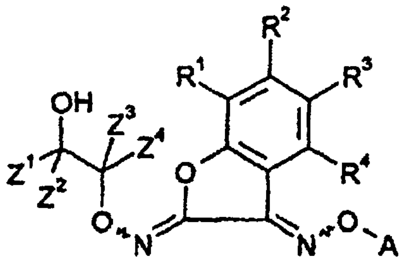

(II),

in which

A, $R^1$, $R^2$, $R^3$, $R^4$, $Z^1$, $Z^2$, $Z^3$ and $Z^4$ are each as defined above,

are rearranged, if appropriate in the presence of a diluent and, if appropriate, in the presence of an acid or a base.

**2.** Process according to Claim 1, wherein in the formula (I)

A represents methyl, ethyl, n- or i-propyl,

$R^1$, $R^2$, $R^3$ and $R^4$ are identical or different and each represent independently of one another hydrogen, halogen, cyano or nitro, or represent alkyl, alkoxy, alkylthio, alkylsulphinyl or alkylsulphonyl, each of which is optionally substituted by 1 to 5 halogen atoms and each of which has 1 to 6 carbon atoms,

$Z^1$, $Z^2$, $Z^3$ and $Z^4$ are identical or different and each represent independently of one another hydrogen, alkyl or hydroxyalkyl having 1 to 4 carbon atoms or halogenoalkyl having 1 to 4 carbon atoms and 1 to 5 identical or different halogen atoms, or

$Z^1$ and $Z^2$ or $Z^1$ and $Z^3$ or $Z^3$ and $Z^4$ form together with the respective carbon atoms that they are attached to a cycloaliphatic ring having five, six or seven carbon atoms.

**3.** Process according to Claim 1, wherein in the formula (I)

A represents methyl or ethyl,

$R^1$, $R^2$, $R^3$ and $R^4$ are identical or different and each represent independently of one another hydrogen, fluorine, chlorine, bromine, cyano, nitro, methyl, ethyl, n- or i-propyl, n-, i-, s- or t-butyl, methoxy, ethoxy, n- or i- propoxy, methylthio, ethylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, and

$Z^1$, $Z^2$, $Z^3$ and $Z^4$ are identical or different and each represent independently of one another hydrogen, methyl, ethyl, n- or i-propyl, n-, i-, s- or t-butyl, hydroxymethyl, trifluoromethyl or trifluoroethyl, or

$Z^1$ and $Z^2$ or $Z^1$ and $Z^3$ or $Z^3$ and $Z^4$ form together with the respective carbon atoms that they are attached to a cycloaliphatic ring having five, six or seven carbon atoms.

**4.** Compounds of the formula (II)

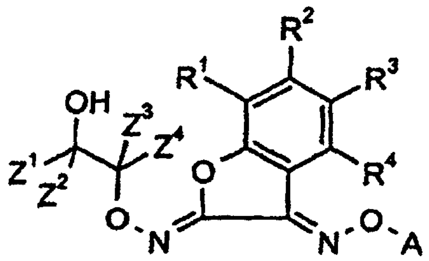

(II),

in which

A, $R^1$, $R^2$, $R^3$, $R^4$, $Z^1$, $Z^2$, $Z^3$ and $Z^4$ are each as defined in Claim 1.

**5.** Process for preparing compounds of the formula (II) as defined in Claim 4, **characterized in that**

d) O-hydroxyethyl-benzofurandione monooximes of the formula

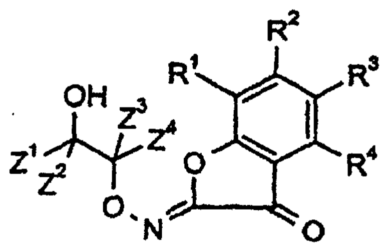

(VII),

in which

$R^1$, $R^2$, $R^3$, $R^4$, $Z^1$, $Z^2$, $Z^3$ and $Z^4$ are each as defined in Claim 1

are reacted with an alkoxyamine of the formula (IV)

$$A\text{-}O\text{-}NH_2 \quad (IV)$$

in which

A is as defined in Claim 1

- or an acid addition complex thereof -
if appropriate in the presence of a diluent and, if appropriate, in the presence of an acid acceptor, or

e) O-alkyl-benzofurandione dioximes of the formula

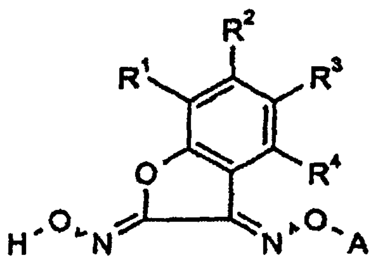

(VIII),

in which

A, $R^1$, $R^2$, $R^3$ and $R^4$ are each as defined in Claim 1,

are reacted with an ethane derivative of the formula

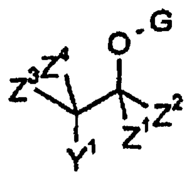

(IX),

in which

$Y^1$ represents halogen, alkylsulphonyloxy, arylsulphonyloxy or alkanoyloxy, and

G represents hydrogen, or

$Y^1$ and G are linked to each other by a single bond, where

$Y^1$ represents oxygen and

G represents

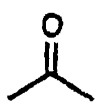

,

or

$Y^1$ and G together represent a single bond, and

$Z^1$, $Z^2$, $Z^3$ and $Z^4$ are each as defined in Claim 1,

if appropriate in the presence of a diluent and, if appropriate, in the presence of a base, or

f) O-hydroxyethyl-benzofurandione dioximes of the formula

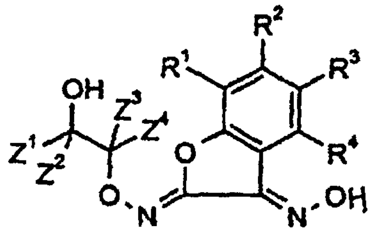

(X),

in which

$R^1$, $R^2$, $R^3$, $R^4$, $Z^1$, $Z^2$, $Z^3$ and $Z^4$ are each as defined in Claim 1,

are reacted with an alkylating agent of the formula (VI)

A-X (VI)

in which

A is as defined in Claim 1, and

X represents halogen, alkylsulphonyloxy, alkoxysulphonyloxy or arylsulphonyloxy,

if appropriate in the presence of a diluent and, if appropriate, in the presence of a base, or

m) O-oxyethyl-O'-methyl-benzofurandione dioximes of the formula

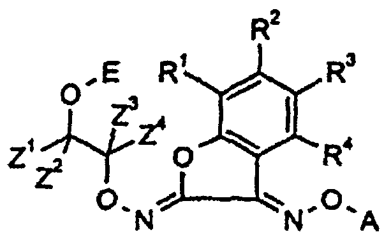

(XIII),

in which

A, $R^1$, $R^2$, $R^3$, $R^4$, $Z^1$, $Z^2$, $Z^3$ and $Z^4$ are each as defined in Claim 1 and

E represents an acyl group or a ketal protecting group,

are reacted with water or an alcohol, if appropriate in the presence of a diluent and, if appropriate, in the presence of an acid or a base.

**6.** Compounds of the formula (VII)

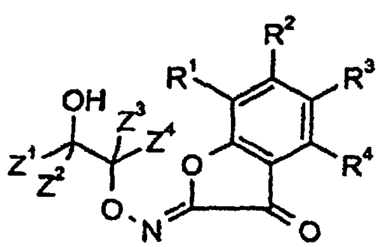

(VII),

in which

$R^1$, $R^2$, $R^3$, $R^4$, $Z^1$, $Z^2$, $Z^3$ and $Z^4$ are each as defined in Claim 1.

**7.** Process for preparing compounds of the formula (VII) as defined in Claim 6, **characterized in that**

g) benzofurandione monooximes of the formula

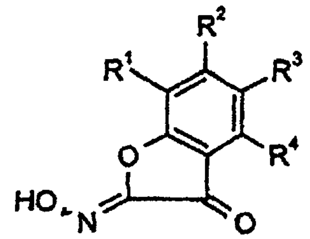

(XI),

in which

$R^1$, $R^2$, $R^3$ and $R^4$ are each as defined in Claim 1,

are reacted with an ethane derivative of the formula (IX),
if appropriate in the presence of a diluent and, if appropriate, in the presence of a base, or

n) O-oxyethyl-benzofurandione monooximes of the formula

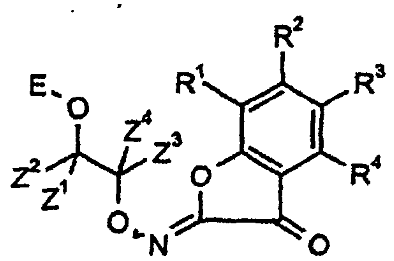

(XIV),

in which

$R^1$, $R^2$, $R^3$, $R^4$, $Z^1$, $Z^2$, $Z^3$ and $Z^4$ are each as defined in Claim 1, and

E is as defined in Claim 5,

are reacted with water or an alcohol, if appropriate in the presence of a diluent and, if appropriate, in the presence of an acid or a base.

**8.** Compounds of the formula (XIV)

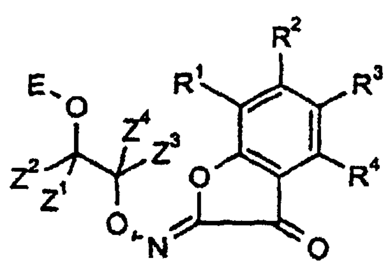

(XIV),

in which

$R^1$, $R^2$, $R^3$, $R^4$, $Z^1$, $Z^2$, $Z^3$ and $Z^4$ are each as defined in Claim 1, and

E represents an acyl group or a ketal protecting group.

**9.** Process for preparing compounds of the formula (XIV) as defined in Claim 8, **characterized in that**

o) benzofurandione monooximes of the formula (XI) are reacted with an ethanol derivative of the formula

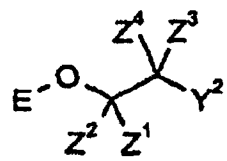

(XV),

in which

E, $Z^1$, $Z^2$, $Z^3$ and $Z^4$ are each as defined in Claim 8, and

$Y^2$ represents halogen, alkylsulphonyloxy, arylsulphonyloxy or alkanoyloxy,

if appropriate in the presence of a diluent and, if appropriate, in the presence of an acid acceptor.

**10.** Compounds of the formula (X)

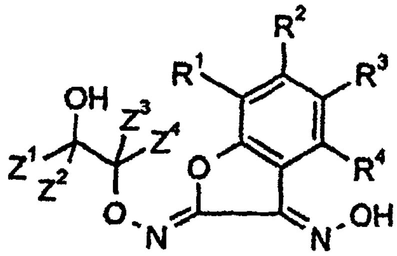

(X),

in which

$R^1$, $R^2$, $R^3$, $R^4$, $Z^1$, $Z^2$, $Z^3$ and $Z^4$ are each as defined in Claim 1.

**11.** Process for preparing compounds of the formula (X) as defined in Claim 10, **characterized in that**

i) O-hydroxyethyl-benzofurandione monooximes of the formula (VII)
are reacted with hydroxylamine - or an acid addition complex thereof -
if appropriate in the presence of a diluent and, if appropriate, in the presence of an acid acceptor, or

q) O-oxyethyl-benzofurandione dioximes of the formula

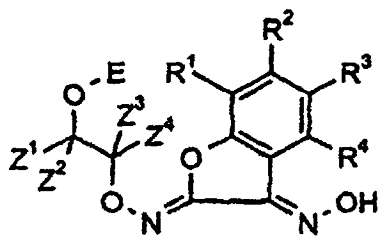

(XVI),

in which

E, $R^1$, $R^2$, $R^3$, $R^4$, $Z^1$, $Z^2$, $Z^3$ and $Z^4$ are each as defined in Claim 10,

are reacted with water or an alcohol, if appropriate in the presence of a diluent and, if appropriate, in the presence of an acid or a base.

**12.** Compounds of the formula (XVI)

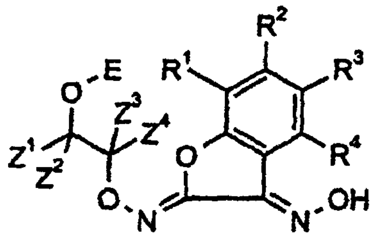

(XVI),

in which

$R^1$, $R^2$, $R^3$, $R^4$, $Z^1$, $Z^2$, $Z^3$ and $Z^4$ are each as defined in Claim 1, and

E represents an acyl group or a ketal protecting group.

**13.** Process for preparing compounds of the formula (XVI) as defined in Claim 12, **characterized in that**

r) O-oxyethyl-benzofurandione monooximes of the formula (XIV) as defined in Claim 7
are reacted with hydroxylamine - or an acid addition complex thereof -
if appropriate in the presence of a diluent and, if appropriate, in the presence of an acid acceptor.

**14.** Compounds of the formula (XIII)

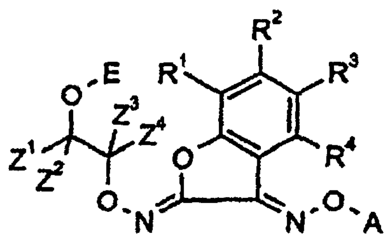

(XIII),

in which

A, $R^1$, $R^2$, $R^3$, $R^4$, $Z^1$, $Z^2$, $Z^3$ and $Z^4$ are each as defined in Claim 1, and

E represents an acyl group or a ketal protecting group.

**15.** Process for preparing compounds of the formula (XIII) as defined in Claim 14, **characterized in that**

s) O-oxyethyl-benzofurandione monooximes of the formula (XIV) as defined in Claim 7 are reacted with an alkoxyamine of the formula (IV) as defined in Claim 5
- or an acid addition complex thereof -
if appropriate in the presence of a diluent and, if appropriate, in the presence of an acid acceptor, or

t) O-oxyethyl-benzofurandione dioximes of the formula (XVI) as defined in Claim 12 are reacted with an alkylating agent of the formula (VI)

A-X (VI)

A is as defined in Claim 1, and

X represents halogen, alkylsulphonyloxy, alkoxysulphonyloxy or arylsulphonyloxy,

if appropriate in the presence of a diluent and, if appropriate, in the presence of a base, or

u) O-alkyl-benzofurandione dioximes of the formula (VIII) as defined in Claim 5 are reacted with an ethanol derivative of the formula (XV) as defined in Claim 9,
if appropriate in the presence of a diluent and, if appropriate, in the presence of an acid acceptor.

**16.** Use of the compounds of the formulae (II), (VII), (XIV), (X), (XVI) and (XIII) as defined in Claims 1, 4, 7, 10, 12 and 14 as intermediates for preparing compounds having fungicidal properties.

**17.** Compound of the formula (II-1)

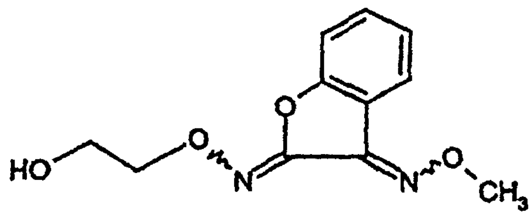

(II-1)

**Revendications**

**1.** Procédé de production de composés de formule (I)

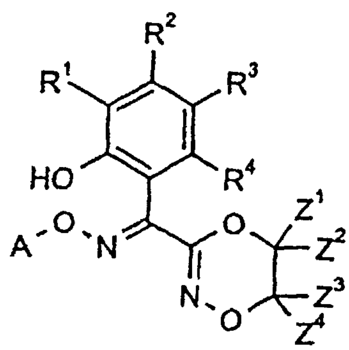

(I),

dans laquelle

| | |
|---|---|
| A | est un groupe alkyle, |
| $R^1$, $R^2$, $R^3$ et $R^4$ | sont identiques ou différents et représentent indépendamment les uns des autres l'hydrogène, un halogène, un groupe cyano, nitro, un groupe alkyle, alkoxy, alkylthio, alkylsulfinyle ou alkylsulfonyle dont chacun est éventuellement substitué par un halogène et |
| $Z^1$, $Z^2$, $Z^3$ et $Z^4$ | sont identiques ou différents et représentent indépendamment les uns des autres l'hydrogène, un groupe alkyle, halogénalkyle ou hydroxyalkyle, ou bien |
| $Z^1$ et $Z^2$ ou $Z^1$ et $Z^3$ ou $Z^3$ et $Z^4$ | forment un noyau cycloaliphatique conjointement avec les atomes de carbone auxquels ils sont liés, |

**caractérisé en ce que** :

a) on transpose des O-hydroxyéthyl-O'-méthylbenzofuranne-dionedioximes de formule

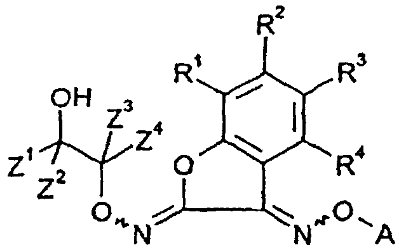

(II),

dans laquelle

A, $R^1$, $R^2$, $R^3$, $R^4$, $Z^1$, $Z^2$, $Z^3$ et $Z^4$ ont les définitions indiquées ci-dessus,

éventuellement en présence d'un diluant et le cas échéant en présence d'un acide ou d'une base.

**2.** Procédé suivant la revendication 1, dans lequel, dans la formule (I)

| | |
|---|---|
| A | est un groupe méthyle, éthyle, n-propyle ou isopropyle, |
| $R^1$, $R^2$, $R^3$ et $R^4$ | sont identiques ou différents et représentent indépendamment les uns des autres l'hydrogène, un halogène, un groupe cyano, nitro, un groupe alkyle, alkoxy, alkylthio, alkylsulfinyle ou alkylsulfonyle ayant chacun 1 à 6 atomes de carbone et chacun étant éventuellement substitué par 1 à 5 atomes d'halogènes, |
| $Z^1$, $Z^2$, $Z^3$ et $Z^4$ | sont identiques ou différents et représentent, indépendamment les uns des autres, l'hydrogène, un groupe alkyle ou hydroxyalkyle ayant 1 à 4 atomes de carbone ou un groupe halogénalkyle ayant 1 à 4 atomes de carbone et 1 à 5 atomes d'halogènes identiques ou différents, ou bien |
| $Z^1$ et $Z^2$ ou $Z^1$ et $Z^3$ ou $Z^3$ et $Z^4$ | forment, conjointement avec les atomes de carbone auxquels ils sont liés, un noyau cycloaliphatique ayant 5, 6 ou 7 atomes de carbone. |

**3.** Procédé suivant la revendication 1, dans lequel, dans la formule (I)

| | |
|---|---|
| A | est un groupe méthyle ou éthyle, |
| $R^1$, $R^2$, $R^3$ et $R^4$ | sont identiques ou différents et représentent, indépendamment les uns des autres l'hydrogène, le fluor, le chlore, le brome, un groupe cyano, nitro, méthyle, éthyle, n-propyle, isopropyle, n-butyle, isobutyle, sec-butyle, tertio-butyle, méthoxy, éthoxy, n-propoxy, isopropoxy, méthylthio, éthylthio, méthyl-sulfinyle, éthylsulfinyle, méthyl-sulfonyle, éthylsulfonyle, trifluoro-méthyle, trifluoréthyle, difluoro-méthoxy, trifluorométhoxy, difluorochlorométhoxy, trifluoréthoxy, difluorométhylthio, difluoro-chlorométhylthio, trifluorométhylthio, trifluoro-méthylsulfinyle ou tri-fluorométhylsulfonyle et |
| $Z^1$, $Z^2$, $Z^3$ et $Z^4$ | sont identiques ou différents et représentent, indépendamment les uns des autres, l'hydrogène, un groupe méthyle, éthyle, n-propyle, isopropyle, n-butyle, isobutyle, secbutyle, tertio-butyle, hydroxyméthyle, trifluoro-méthyle ou trifluoréthyle, ou bien |
| $Z^1$ et $Z^2$ ou $Z^1$ et $Z^3$ ou $Z^3$ et $Z^4$ | forment, conjointement avec les atomes de carbone auxquels ils sont liés, un noyau cyclo-aliphatique ayant 5, 6 ou 7 atomes de carbone. |

**4.** Composés de formule (II)

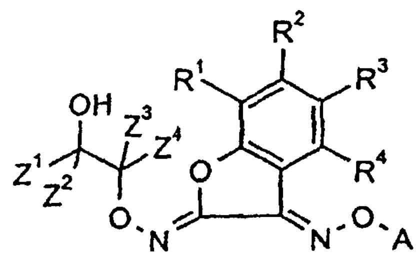

(II),

dans laquelle

A, $R^1$, $R^2$, $R^3$, $R^4$, $Z^1$, $Z^2$, $Z^3$ et $Z^4$ ont les définitions indiquées dans la revendication 1.

**5.** Procédé de production de composés de formule (II) telle que définie dans la revendication 4, **caractérisé en ce que** :

d) on fait réagir des O-hydroxyéthylbenzofurannedionemono-oximes de formule

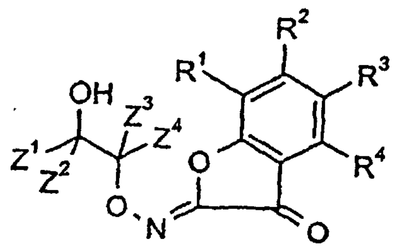

(VII),

dans laquelle

$R^1$, $R^2$, $R^3$, $R^4$, $Z^1$, $Z^2$, $Z^3$ et $Z^4$ ont les définitions indiquées dans la revendication 1,

avec une alkoxyamine de formule (IV)

$$A\text{-}O\text{-}NH_2 \quad (IV),$$

dans laquelle A a la définition indiquée dans la revendication 1
- ou avec un complexe d'addition d'acide de ce composé -
le cas échéant en présence d'un diluant et en présence éventuelle d'un accepteur d'acide, ou **en ce que** :
e) on fait réagir des O-alkylbenzofurannedionedioximes de formule

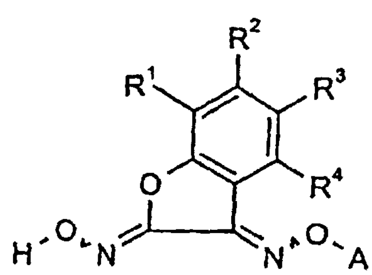

(VIII),

dans laquelle

A, $R^1$, $R^2$, $R^3$, et $R^4$ ont les définitions indiquées dans la revendication 1,

avec un dérivé d'éthane de formule

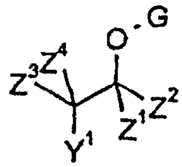

(IX),

dans laquelle

$Y^1$ représente un halogène, un groupe alkylsulfonyloxy, arylsulfonyloxy ou alcanoyloxy et
G représente l'hydrogène, ou bien
$Y^1$ et G sont liés l'un à l'autre par une liaison simple,

où

$Y^1$ représente l'oxygène ou bien
G est un groupe

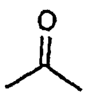

ou bien
$Y^1$ et G forment ensemble une liaison simple et

$Z^1$, $Z^2$, $Z^3$ et $Z^4$ ont les définitions indiquées dans la revendication 1,

le cas échéant en présence d'un diluant et en présence éventuelle d'une base, ou **en ce que** :
f) on fait réagir des O-hydroxyéthylbenzofurannedionedioximes de formule

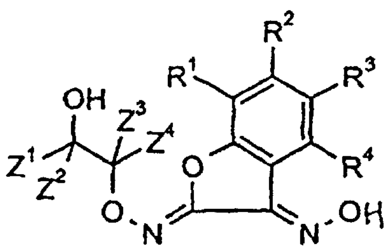

(X),

dans laquelle

$R^1$, $R^2$, $R^3$, $R^4$, $Z^1$, $Z^2$, $Z^3$ et $Z^4$ ont les définitions indiquées dans la revendication 1,

avec un agent d'alkylation de formule (VI)

$$A\text{-}X \qquad (VI),$$

dans laquelle A a la définition indiquée dans la revendication 1 et X représente un halogène, un groupe alkylsulfonyloxy, alkoxysulfonyloxy ou arylsulfonyloxy,
le cas échéant en présence d'un diluant et en présence éventuelle d'une base,
ou **en ce que**
m) on fait réagir des O-oxyéthyl-O'-méthylbenzofurannedionedioximes de formule

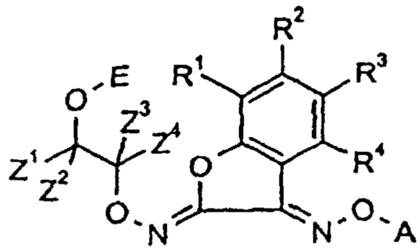

(XIII),

dans laquelle

A, $R^1$, $R^2$, $R^3$, $R^4$, $Z^1$, $Z^2$, $Z^3$ et $Z^4$ ont les définitions indiquées dans la revendication 1 et
E est un groupe acyle ou un groupe protecteur du type cétal,

avec l'eau ou un alcool, éventuellement en présence d'un diluant et le cas échéant en présence d'un acide ou d'une base.

**6.** Composés de formule (VII)

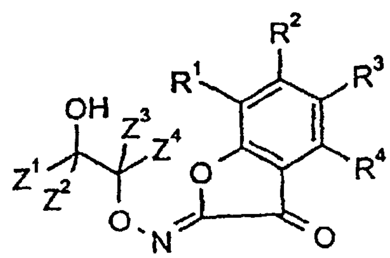

(VII),

dans laquelle

$R^1$, $R^2$, $R^3$, $R^4$, $Z^1$, $Z^2$, $Z^3$ et $Z^4$ ont les définitions indiquées dans la revendication 1,

**7.** Procédé de production de composés de formule (VII) telle que définie dans la revendication 6, **caractérisé en ce que** :

g) on fait réagir des benzofurannedione-monooximes de formule

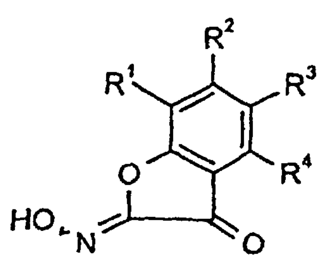

(XI),

dans laquelle

$R^1$, $R^2$, $R^3$ et $R^4$ ont les définitions indiquées dans la revendication 1,

avec un dérivé d'éthane de formule (IX),
le cas échéant en présence d'un diluant et en présence éventuelle d'une base, ou **en ce que** :
n) on fait réagir des O-oxyéthylbenzofurannedionemonooximes de formule

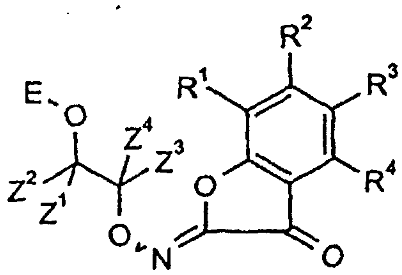

(XIV),

dans laquelle

| | |
|---|---|
| $R^1$, $R^2$, $R^3$, $R^4$, $Z^1$, $Z^2$, $Z^3$ et $Z^4$ | ont les définitions indiquées dans la revendication 1, et |
| E | a les définitions indiquées dans la revendication 5, |

avec l'eau ou un alcool, éventuellement en présence d'un diluant et le cas échéant en présence d'un acide ou d'une base.

**8.** Composés de formule (XIV)

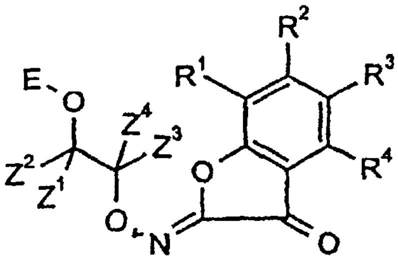

(XIV),

dans laquelle

| | |
|---|---|
| $R^1$, $R^2$, $R^3$, $R^4$, $Z^1$, $Z^2$, $Z^3$ et $Z^4$ | ont les définitions indiquées dans la revendication 1, et |
| E | représente un groupe acyle ou un groupe protecteur du type cétal. |

**9.** Procédé de production de composés de formule (XIV) telle que définie dans la revendication 8, **caractérisé en ce que** :

o) on fait réagir des benzofurannedione-monooximes de formule (XI) avec un dérivé d'éthanol de formule

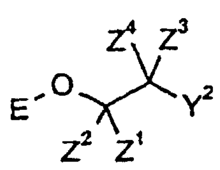

(XV),

dans laquelle

| | |
|---|---|
| E, $Z^1$, $Z^2$, $Z^3$ et $Z^4$ | ont les définitions indiquées dans la revendication 8, et |
| $Y^2$ | représente un halogène, un groupe alkylsulfonyloxy, arylsulfonyloxy ou alcanoyloxy, |

le cas échéant en présence d'un diluant et en présence éventuelle d'un accepteur d'acide.

**10.** Composés de formule (X)

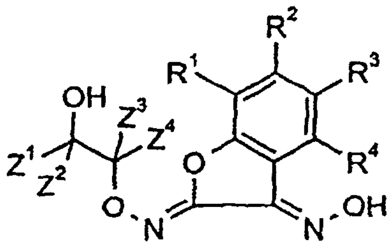

(X),

dans laquelle

$R^1$, $R^2$, $R^3$, $R^4$, $Z^1$, $Z^2$, $Z^3$ et $Z^4$ ont les définitions indiquées dans la revendication 1.

**11.** Procédé de production de composés de formule (X) telle que définie dans la revendication 10, **caractérisé en ce que** :

i) on fait réagir des O-hydroxyéthylbenzofurannedionemonooximes de formule (VII) avec l'hydroxylamine - ou un complexe d'addition d'acide de ce composé - éventuellement en présence d'un diluant et en présence éventuelle d'un accepteur d'acide, ou **en ce que**
q) on fait réagir des O-oxyéthylbenzofurannedionedioximes de formule

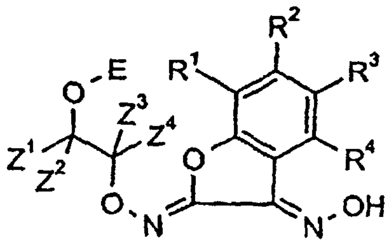

(XVI),

dans laquelle

E, $R^1$, $R^2$, $R^3$, $R^4$, $Z^1$, $Z^2$, $Z^3$ et $Z^4$ ont les définitions indiquées dans la revendication 10,

avec l'eau ou un alcool, éventuellement en présence d'un diluant et le cas échéant en présence d'un acide ou d'une base.

**12.** Composés de formule (XVI)

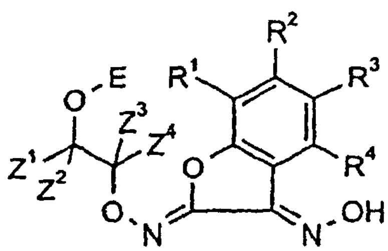

(XVI),

dans laquelle

$R^1$, $R^2$, $R^3$, $R^4$, $Z^1$, $Z^2$, $Z^3$ et $Z^4$ ont les définitions indiquées dans la revendication 1 et
E représente un groupe acyle ou un groupe protecteur du type cétal.

**13.** Procédé de production de composés de formule (XVI) telle que définie dans la revendication 12, **caractérisé en ce que** :

r) on fait réagir des O-oxyéthylbenzofurannedionemonooximes de formule (XIV) telle que définie dans la revendication 7
avec l'hydroxylamine - ou un complexe d'addition d'acide de ce composé -
le cas échéant en présence d'un diluant et en présence éventuelle d'un accepteur d'acide.

**14.** Composés de formule (XIII)

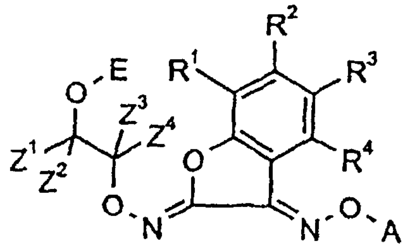

(XIII),

dans laquelle

A, $R^1$, $R^2$, $R^3$, $R^4$, $Z^1$, $Z^2$, $Z^3$ et $Z^4$ ont les définitions indiquées dans la revendication 1 et
E représente un groupe acyle ou un groupe protecteur du type cétal.

**15.** Procédé de production de composés de formule (XIII) telle que définie dans la revendication 14, **caractérisé en ce que** :

(s) on fait réagir des O-oxyéthylbenzofurannedionemonooximes de formule (XIV) telle que définie dans la revendication 7, avec une alkoxyamine de formule (IV) telle que définie dans la revendication 5,
- ou un complexe d'addition d'acide de ce composé -
le cas échéant en présence d'un diluant et en présence éventuelle d'un accepteur d'acide, ou **en ce que** :
t) on fait réagir des O-oxyéthylbenzofurannedionedioximes de formule (XVI) telle que définie dans la revendication 12, avec un agent d'alkylation de formule (VI)

$$A - X \qquad (VI)$$

dans laquelle

A a la définition indiquée dans la revendication 1 et
X représente un halogène, un groupe alkylsulfonyloxy, alkoxysulfonyloxy ou arylsulfonyloxy,

le cas échéant en présence d'un diluant et en présence éventuelle d'une base, ou **en ce que**
u) on fait réagir des O-alkylbenzofurannedionedioximes de formule (XIII) telle que définie dans la revendication 5, avec un dérivé d'éthanol de formule (XV) telle que définie dans la revendication 9, le cas échéant en présence d'un diluant et en présence éventuelle d'un accepteur d'acide.

**16.** Utilisation des composés de formules (II), (VII), (XIV), (X), (XVI) et (XIII) telles que définies dans les revendications 1, 4, 7, 10, 12 et 14 comme produits intermédiaires ou comme précurseurs, pour l'obtention de composés doués de propriétés fongicides.

**17.** Composé de formule (II-1)

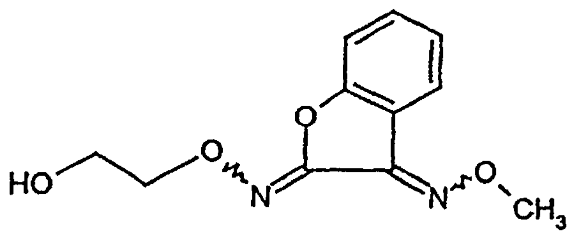

(II-1)